# EUROPÄISCHE PATENTSCHRIFT

(11) **EP 0 956 294 B1**
(45) Veröffentlichungstag und Bekanntmachung des Hinweises auf die Patenterteilung: **09.06.2004**
(21) Anmeldenummer: 97936672.1
(22) Anmeldetag: 29.07.1997
(51) Int. Cl.: C07K 5/06, A61K 38/05

(54) **THROMBININHIBITOREN**
THROMBIN INHIBITORS
INHIBITEURS DE LA THROMBINE

(30) Priorität: 14.08.1996 DE 19632773
(43) Veröffentlichungstag der Anmeldung: 17.11.1999
(73) Patentinhaber: Abbott GmbH & Co. KG, 65205 Wiesbaden (DE)
(72) Erfinder: BAUCKE, Dorit, D-68163 Mannheim (DE); LANGE, Udo, D-67063 Ludwigshafen (DE); MACK, Helmut, D-67067 Ludwigshafen (DE); SEITZ, Werner, D-68723 Plankstadt (DE); ZIERKE, Thomas, D-67459 Böhl-Iggelheim (DE); HÖFFKEN, Hans, Wolfgang, D-67069 Ludwigshafen (DE); HORNBERGER, Wilfried, D-67434 Neustadt (DE)
(74) Vertreter: Grünecker, Kinkeldey, Stockmair & Schwanhäusser Anwaltssozietät
(86) Internationale Anmeldenummer: PCT/EP1997/004104
(87) Internationale Veröffentlichungsnummer: WO 1998/006741

(56) Entgegenhaltungen:
- EP-A- 0 672 658

## Beschreibung

Die vorliegende Erfindung betrifft neue fünfgliedrige heterocyclische Amidine, ihre Herstellung und ihre Verwendung als kompetitive Inhibitoren von Trypsin-ähnlichen Serinproteasen, besonders Thrombin und Kininogenasen wie Kallikrein. Die Erfindung bezieht sich auch auf pharmazeutische Zusammensetzungen, die die Verbindungen als aktive Bestandteile enthalten, sowie die Verwendung der Verbindungen als Thrombininhibitoren, Antikoagulantien und als antiinflammatorische Agenzien.

Thrombin gehört zur Gruppe der Serinproteasen und spielt als terminales Enzym in der Blutgerinnungskaskade eine zentrale Rolle. Sowohl die intrinsische als auch die extrinsische Gerinnungskaskade führen über mehrere Verstärkungsstufen zur Entstehung von Thrombin aus Prothrombin. Die thrombinkatalysierte Spaltung von Fibrinogen zu Fibrin leitet dann die Blutgerinnung und die Aggregation der Thrombozyten ein, die ihrerseits durch die Bindung von Plättchenfaktor 3 und Gerinnungsfaktor XIII sowie eine ganze Reihe von hochaktiven Mediatoren die Thrombinbildung verstärken.

Thrombinbildung und -wirkung sind zentrale Ereignisse bei der Entstehung sowohl von weißen, arteriellen als auch von roten, venösen Thromben und daher potentiell wirksame Angriffspunkte für Pharmaka. Thrombininhibitoren sind im Gegensatz zu Heparin in der Lage, unabhängig von Kofaktoren gleichzeitig die Wirkungen von freiem Thrombin als auch an Thrombozyten gebundenes vollständig zu hemmen. Sie können in der Akutphase thromboembolische Ereignisse nach perkutaner transluminaler koronarer Angioplastie (PTCA) und Lyse verhindern und als Antikoagulantien in der extrakorporalen Zirkulation (Herz-Lungen-Maschine, Hämodialyse) dienen. Sie können auch allgemein zur Thromboseprophylaxe, beispielsweise nach chirurgischen Eingriffen dienen.

Es ist bekannt, daß synthetische Argininderivate die Enzymaktivität des Thrombins beeinflussen, indem sie mit dem aktiven Serinrest der Protease Thrombin in Wechselwirkung treten. Peptide auf der Basis Phe-Pro-Arg, in denen die N-terminale Aminosäure in der D-Form vorliegt, haben sich als besonders günstig erwiesen. D-Phe-Pro-Arg-isopropylester ist als kompetitiv wirkender Thrombininhibitor beschrieben (C.Mattson u.a., Folia Haematol, 109, 43 bis 51, 1983).

Die Derivatisierung des C-Terminus Arginin zum Aldehyd führt zu einer Verstärkung der Inhibitorwirkung. So sind eine Vielzahl von Arginalen beschrieben, die die Hydroxylgruppe des "aktiven" Serins halbacetalisch zu binden vermögen (EP 185390, 479489, 526877, 542525; WO 93/15756, 93/18060.

Die thrombininhibitorische Wirksamkeit peptidischer Ketone, fluorierter Alkylketone, sowie von Ketoestern, Borsäurederivaten, Phosphorsäureestern und α-Ketocarbonsäureamiden ist ebenfalls mit dieser Serin-Wechselwirkung erklärbar (EP 118280, 195212, 362002, 364344, 410411, 471651, 589741, 293881, 503203, 504064, 530167; WO 92/07869, 94/08941).

Bei den von J. Oleksyszyn u.a. in J. Med. Chem. 37, 226 bis 231 (1994) beschriebenen peptidischen 4-Amidinophenyl-glycinphosphonat-diphenylestern handelt es sich um irreversible Thrombininhibitoren mit unzureichender Selektivität gegenüber a deren Serinproteasen.

In DE 3 108 810, WO 93/11152 und EP 601 459 sind Agmatin und damit Arginin-Derivate beschrieben, die keine Wechselwirkung mit dem aktiven Serin der Serinproteasen eingehen können.

WO 94/29336, EP 0 601 459 und WO 95/23609 stellen eine Weiterentwicklung dar, wobei der Agmatin- durch einen Arylamidinrest ersetzt ist.

Kininogenasen sind Serinproteasen, die aus Kininogenen vasoaktive Peptide, die sog. Kinine (Bradykinin, Kallidin und Met-Lysbradykinin), freisetzen. Kininogene stellen multifunktionale Proteine dar, die in Kaskadenreaktionen der Gerinnung und Entzündung auftreten. Als Inhibitoren schützen sie Zellen vor der Zerstörung durch Cystein-Proteasen (Müller Esterl, 1985, FEBS Lett. 182, 310-314). Wichtige Kininogenasen sind Plasma-Kallikrein, Gewebs-Kallikrein und Mastzellen-Tryptase.

Kinine wie Bradykinin und Kallidin sind vasoaktive Peptide, die eine Vielzahl biologischer Prozesse beeinflussen. Sie spielen in entzündlichen Prozessen eine wesentliche Rolle. Durch Erhöhung der vaskulären Permeabilität führen sie zu Hypotension und Ödemen. Weiterhin sind sie sehr potente schmerzproduzierende Antacoide und haben als zelluläre Mediatoren in der Pathophysiologie des Asthmas, der allergischen Rhinitis und der Arthritis große Bedeutung (K.D. Bhoola, C.D. Figueroa, K. Worthy, Pharmacological Revies 1992, 44 (1), 1-80).

Unabhängig von den Mechanismen, die entzündlichen Prozessen zugrundeliegen, kommt es zum Austritt von Flüssigkeit aus den Blutgefäßen, die alle Protein-Systeme des zirkulierenden Blutes enthält. Das bedeutet, daß der Austritt von Plasmaflüssigkeit aus den Gefäßen in Krankheiten wie Asthma, Rhinitis und entzündungsbedingten inneren Krankheiten eine Rolle spielt. Besonders in allergischen Prozessen wird dabei Mastzell-Tryptase freigesetzt (Salomonsson et al., Am. Rev. Respir. Dis., 1992, 146, 1535-1542).

Die Arginin-Chloromethylketone H-(D)-Pro-Phe-Arg-CH₂Cl und H-(D)-Phe-Phe-Arg-CH₂-Cl wurden von Kettner und Shaw als Plasma-Kallikreininhibitoren beschrieben (Biochem. 1978, 17, 4778-4784 und Meth. Enzym. 1981, 80, 826-842).

Verschiedene synthetische Derivate von Benzamidinen und Benzylaminen erwiesen sich als Inhibitoren von Plasmakallikrein, wobei die Benzamidine eine wesentlich stärkere inhibitorische Wirkung aufwiesen (F. Markward, S. Drawert, P. Walsmann, Biochemical Pharmacology 1974, 23, 2247-2256).

Auch PKSI-527, das Hydrochlorid von N-(trans-4-aminomethylcyclohexylcarbonyl)-L-phenylalanin-4-carboxymethyl-anilid, ist ein wirksamer Inhibitor für diese Kininogenase (Wanaka, Ohamoto et al., Thromb. Res. 1990, 57 (6), 889-895).

Gegenstand der Erfindung sind Verbindungen der Formel I 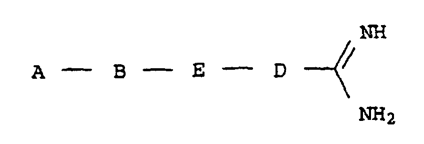 worin A, B, D und E folgende Bedeutung besitzen:
A: 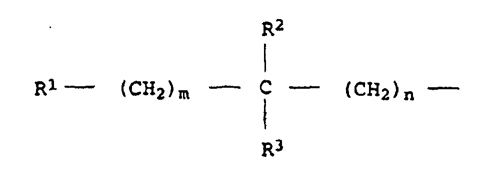 worin
   - m: 0, 1 oder 2,
   - n: 0, 1 oder 2,
   - R¹: HOOC-, C₁₋₆-Alkyl-OOC- oder -OH,
   - R²: H-, C₁₋₄-Alkyl- oder R¹-(CH₂)ₘ-,
   - R³: H- oder C₁₋₄-Alkyl-,
   darstellen,
B: 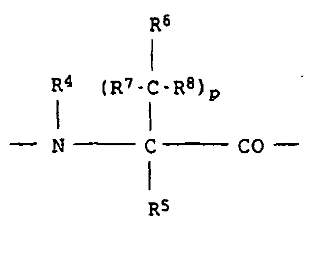 worin
   - R⁴: H-, C₁₋₄-Alkyl- oder R¹-(CH₂)ₘ- (wobei R¹ und m die oben angegebene Bedeutung besitzen),
   - p: 0 oder 1,
   - R⁵: H- oder C₁₋₄-Alkyl-,
   - R⁶: H-, C₁₋₈-Alkyl-, Phenyl-, welches bis zu drei gleiche oder verschiedene Reste der Gruppe C₁₋₄-Alkyl-, CF₃-, C₁₋₄-Alkoxy-, F- oder Cl- tragen kann, C₃₋₈-Cycloalkyl-, welches bis zu vier gleiche oder verschiedene C₁₋₄-Alkylreste tragen kann oder wobei eine oder zwei C-C-Einfachbindungen im Ring durch eine C=C-Doppelbindung ersetzt sein können, oder ein Phenylring ankondensiert sein kann, C₇-C₁₂-Bicycloalkyl- oder C₁₀-Tricycloalkyl- oder
   - R⁴ und R⁶: zusammen eine Ethylen- oder Propylengruppe,
   - R⁷: H, C₁₋₈-Alkyl-, Phenyl-, welches bis zu drei gleiche oder verschiedene Reste der Gruppe C₁₋₄-Alkyl-, CF₃-, C₁₋₄-Alkoxy-, F- oder Cl- tragen kann, C₃₋₈-Cycloalkyl-, welches bis zu vier gleiche oder verschiedene C₁₋₄-Alkylreste tragen kann,
   - R⁸: H oder C₁₋₄-Alkyl,
E: 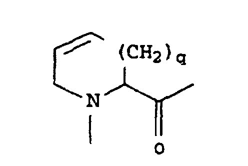 q = 0 oder 1
D: 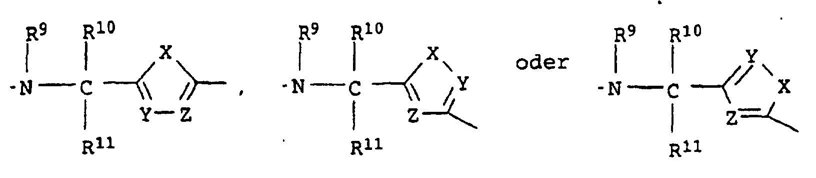 worin
   - R⁹: H- oder C₁₋₃-Alkyl-,
   - R¹⁰: H- oder C₁₋₄-Alkyl-,
   - R¹¹: H- oder C₁₋₄-Alkyl-,
   - X: O, S, -NR¹² (R¹² = H-, C₁₋₆-Alkyl-),
   - Y: -N= oder -CR¹³= (R¹³ = H-, C₁₋₄-Alkyl-, Cl, CF₃),
   - Z: -N= oder -CR¹³= bedeuten,
sowie deren Salze mit physiologisch verträglichen Säuren.

Die durch B dargestellten Aminosäurederivate sind vorzugsweise (D)-konfiguriert, das 3,4-Dehydroprolin bzw. die 4,5-Dehydropipecolinsäure (L)-konfiguriert.

Bevorzugt sind Verbindungen der Formel I, worin die Gruppen A bis E folgende Bedeutung besitzen:
A
   HOOC - (CH₂)ₜ - (t = 1, 2 oder 3) , (HOOC-CH₂)₂-CH-, (HO-CH₂)₂CH-,
   HOOC-CH₂-CH(COOH)-, HOOC-CH(CH₂-CH₂-OH) -, HOOC-CH(C₁₋₄-Alkyl)-,
   HOOC-C(C₁₋₄-Alkyl)₂-, C₁₋₄-Alkyl-OOC-(CH₂)ₜ-,
B 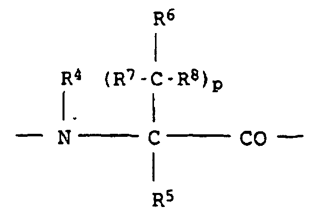
   - p: 0, 1,
   - R⁴: H-, C₁₋₄-Alkyl- oder HOOC-(CH₂)ₘ- (m = 1, 2 oder 3),
   - R⁵: H-, Methyl-
   - R⁶: H-, C₁₋₈-Alkyl-, Phenyl-, welches bis zu drei gleiche oder verschiedene Reste der Gruppe CH₃-, CF₃-, CH₃-O-, F- oder Cl-tragen kann, C₃₋₈-Cycloalkyl-, welches bis zu vier Methylreste tragen kann, Cyclohexa-1,4-dienyl-, Bicyclo[2.2.2]-octyl-, Bicyclo[2.2.1]-heptyl-, Norbornyl-, Adamantyl-, Indanyl-, Decalinyl-,
   - R⁷: H, C₁₋₈-Alkyl-, Phenyl-, welches bis zu drei gleiche oder verschiedene Reste der Gruppe CH₃-, CF₃-, CH₃O-, F- oder Cl- tragen kann, C₃₋₈-Cycloalkyl-, welches bis zu vier Methylreste tragen kann,
   - R⁸: H, C₁₋₄-Alkyl,
   (Der Baustein B ist vorzugsweise D-konfiguriert),
E 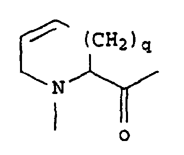
   - q: 0, 1
   (Der Baustein E ist vorzugsweise L-konfiguriert) ,
D 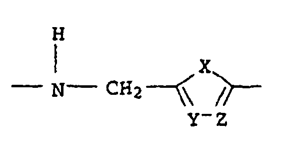 mit
   X = S, O, NH, NCH₃, NC₂H₅,
   Y = CH, C-CH₃, C-Cl, C-CF₃ und
   Z = CH, C-CH₃, C-Cl, C-CF₃
   oder X = S, O, NH, N-CH₃ Y = N Z = CH, C-CH₃, C-CF₃
   oder X = S, O, NH, N-CH₃ Y = CH, C-CH₃, C-CF₃ Z = N
   oder X = S, O, NH, N-CH₃ Y = N Z = N 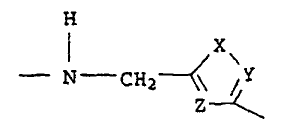 mit
   X = S, O, NH, NCH₃, NC₂H₅,
   Y = CH, C-CH₃, C-CF₃ und
   Z = CH, C-CH₃, C-CF₃, C-Cl
   oder X = O, NH, NCH₃ Y = N Z = CH, C-CH₃, C-CF₃
   oder X = O, S, NH, NCH₃ Y = CH, C-CH₃, Z = N
   C-CF₃
   oder X = O, S, NH, NCH₃ Y = Z = N 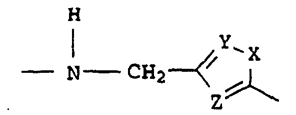 mit
   X = S, O, NH, NCH₃, NC₂H₅,
   Y = CH, C-CH₃, C-CF₃ und
   Z = CH, C-CH₃, C-CF₃, C-Cl
   oder X = O, NH, NCH₃ Y = N Z = CH, C-CH₃,
   C-CF₃, C-Cl
   oder X = O, S, NH, NCH₃ Y = CH, C-CH₃, Z = N
   C-CF₃
   oder X = O, NH, NCH₃ Y = Z = N.

Besonders bevorzugt sind Verbindungen der Formel I, in denen A, B, D und E folgende Bedeutung besitzen
A HOOC-CH₂, HOOC-CH₂-CH₂, HOOC-CH(CH₃), HOOC-CH(C₂H₅)
B 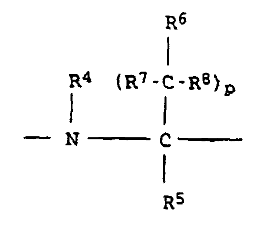
   - p: 0, 1,
   - R⁴: H-, CH₃-
   - R⁵: H-, CH₃-,
   - R⁶: C₁₋₈-Alkyl-, C₅₋₈-Cycloalkyl-, welches bis zu vier Methylreste tragen kann, Bicyclo[2.2.2]octyl, Bicylo[2.2.1]heptyl, Norbornyl, Adamantyl, Indanyl, Decalinyl, wobei Cyclopentyl-, Cyclohexyl- und Cycloheptyl- besonders bevorzugt sind,
   - R⁷: H, CH₃-,
   - R⁸: H, CH₃-,
E 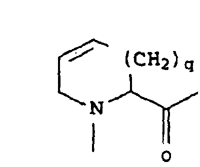
   - q: 0,1
D 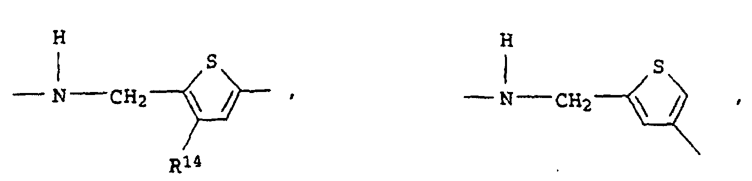 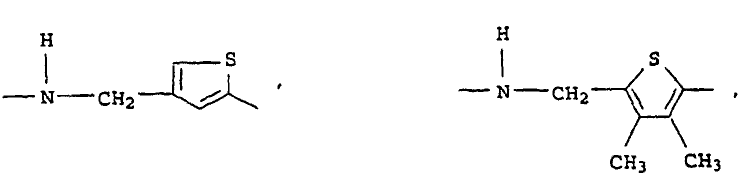 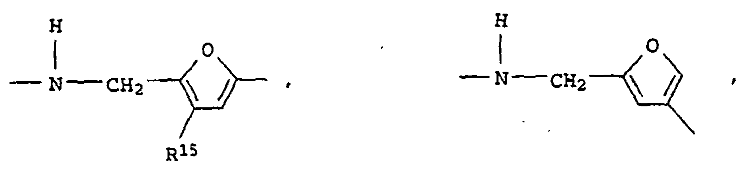 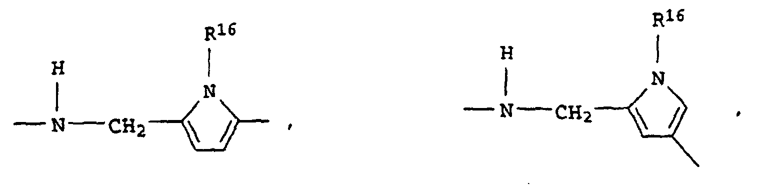 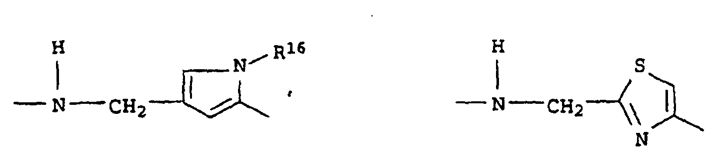 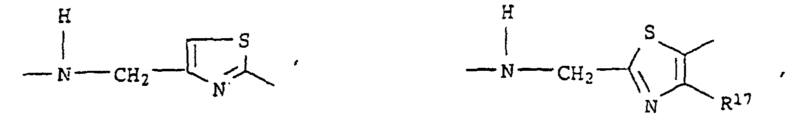 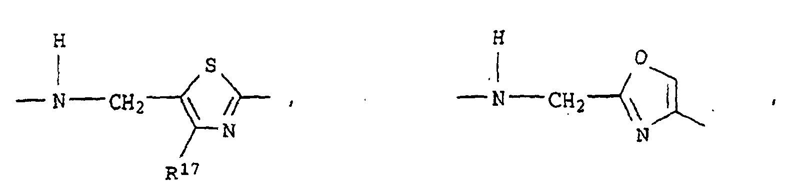 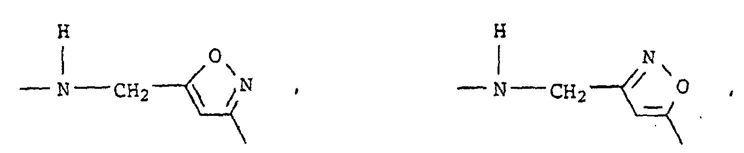 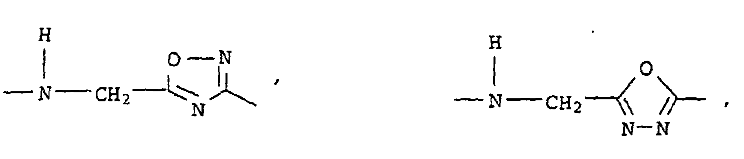 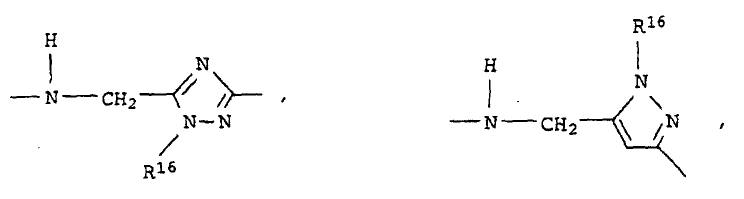 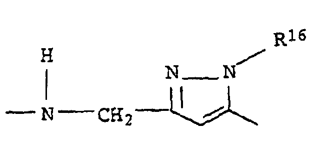 mit
   R¹⁴: H, CH₃, Cl, CF₃, vorzugsweise H
   R¹⁵: H, Cl, CF₃, vorzugsweise H
   R¹⁶: H, CH₃, C₂H₅, vorzugsweise CH₃
   R¹⁷: H, CH₃, CF₃, vorzugsweise H, CH₃

Folgende Substanzen sind besonders bevorzugt:
tBuOOC-CH₂- (D)Chg-Pyr-NH-CH₂-5- (2-am) -thioph
HOOC-CH₂- (D)Chg-Pyr-NH-CH₂-5- (2-am) -thioph
tBuOOC-CH₂-(D)Chg-Dep-NH-CH₂-5-(2-am)-thioph
HOOC-CH₂-(D)Chg-Dep-NH-CH₂-5-(2-am)-thioph
HOOC-CH₂-(D,L)Cpg-Pyr-NH-CH₂-5-(2-am)-thioph
HOOC-CH₂- (D,L)Cheg-Pyr-NH-CH₂-5- (2-am) -thioph
HOOC-CH₂-(D,L)Cog-Pyr-NH-CH₂-5-(2-am)-thioph
HOOC-CH₂-(D,L)Nog-Pyr-NH-CH₂-5-(2-am)-thioph
HOOC-CH₂-(D,L)Adaala-Pyr-NH-CH₂-5-(2-am)-thioph
HOOC-CH₂- (D,L)4-MeCha-Pyr-NH-CH₂-5- (2-am) -thioph
HOOC-CH₂- (D,L)γ-MeCha-Pyr-NH-CH₂-5- (2-am) -thioph
HOOC-CH₂- (D,L)4-MeChg-Pyr-NH-CH₂-5- (2-am) -thioph
HOOC-CH₂- (D,L)3,3-Me₂Chg-Pyr-NH-CH₂-5- (2-am) -thioph
HOOC-CH₂- (D,L) 3,3-Me₂Cha-Pyr-NH-CH₂-5- (2-am) -thioph
HOOC-CH₂- (D,L)4-iPrChg-Pyr-NH-CH₂-5- (2-am) -thioph
HOOC-CH₂- (D,L)3,4,5(MeO)₃Phe-Pyr-NH-CH₂-5- (2-am) -thioph
HOOC-CH₂- (D,L)Chea-Pyr-NH-CH₂-5- (2-am) -thioph
HOOC-CH₂- (D)Diphe-Pyr-NH-CH₂-5- (2-am) -thioph
HOOC-CH₂- (D,L)ββ-Me₂Cha-Pyr-NH-CH₂-5- (2-am) -thioph
HOOC-CH₂- (D,L)Adagly-Pyr-NH-CH₂-5- (2-am) -thioph
HOOC-CH₂- (D,L)-1-Tic-Pyr-NH-CH₂-5- (2-am) -thioph
HOOC-CH₂- (D,L)Dch-Pyr-NH-CH₂-5- (2-am) -thioph
HOOC-CH₂- (D,L)4-iPrCha-Pyr-NH-CH₂-5- (2-am) -thioph
HOOC-CH₂- (D)α-MeCha-Pyr-NH-CH₂-5- (2-am) -thioph
HOOC-CH₂- (D)α-MeCha-Dep-NH-CH₂-5- (2-am) -thioph
HOOC-CH₂- (N-Me)(D)Cha-Pyr-NH-CH₂-5- (2-am) -thioph
HOOC-CH₂- (D)Cha-Pyr-NH-CH₂-5- (2-am) -thioph
HOOC-CH₂- (D)Cha-Dep-NH-CH₂-5- (2-am) -thioph
HOOC-CH₂- (D)Cha-Pyr-NH-CH₂-4- (2-am) -thioph
HOOC-CH₂- (D)Cha-Dep-NH-CH₂-4- (2-am) -thioph
HOOC-CH₂- (D)Cha-Pyr-NH-CH₂-5- (3-am) -thioph
HOOC-CH₂- (D)Cha-Dep-NH-CH₂-5- (3-am) -thioph
HOOC-CH₂- (D)Chg-Pyr-NH-CH₂-5- (3-am) -thioph
HOOC-CH₂- (D)Chg-Pyr-NH-CH₂-4- (2-am) -thioph
HOOC-CH₂- (D)Cha-Pyr-NH-CH₂-5- (2-am) -fur
HOOC-CH₂- (D)Cha-Dep-NH-CH₂-5- (2-am) -fur
HOOC-CH₂- (D)Cha-Pyr-NH-CH₂-4- (2-am) -fur
HOOC-CH₂- (D)Cha-Dep-NH-CH₂-4- (2-am) -fur
HOOC-CH₂- (D)Cha-Pyr-NH-CH₂-5- (3-am) -fur
HOOC-CH₂- (D)Cha-Dep-NH-CH₂-5- (3-am) -fur
HOOC-CH₂ -(D)Chg-Pyr-NH-CH₂-5- (2-am) -fur
HOOC-CH₂- (D)Chg-Pyr-NH-CH₂-5- (3-am) -fur
HOOC-CH₂ -(D)Cha-Pyr-NH-CH₂-5- (1-Me-2-am) -pyrr
HOOC-CH₂ -(D)Chg-Pyr-NH-CH₂-5- (1-Me-2-am) -pyrr
HOOC-CH₂- (D)Cha-Dep-NH-CH₂-5- (1-Me-2-am) -pyrr
HOOC-CH₂- (D)Cha-Pyr-NH-CH₂-4- (1-Me-2-am) -pyrr
HOOC-CH₂- (D)Cha-Dep-NH-CH₂-4-(1-Me-2-am)-pyrr
HOOC-CH₂- (D)Cha-Pyr-NH-CH₂-5- (1-Me-3-am) -pyrr
HOOC-CH₂- (D)Cha-Dep-NH-CH₂-5- (1-Me-3-am) -pyrr
HOOC-CH₂- (D)Cha-Pyr-NH-CH₂-5- (2-am-3,4 -Me₂) -thioph
HOOC-CH₂- (D)Cha-Pyr-NH-CH₂-5- (2-am-3-Me) -thioph
HOOC-CH₂- (D)Cha-Pyr-NH-CH₂-5- (2-am-4-Me) -thioph
HOOC-CH₂- (D)Cha-Pyr-NH-CH₂-5- (2-am-3-Me) -fur
HOOC-CH₂- (D)Cha-Pyr-NH-CH₂-5-(2-am-4-Me)-fur
HOOC-CH₂-(D)Cha-Pyr-NH-CH₂-5-(3-am-2-Me)-fur
HOOC-CH₂- (D)Cha-Pyr-NH-CH₂-2- (5-am) -thiaz
HOOC-CR₂- (D)Cha-Pyr-NR-CH₂-2- (4-am) -thiaz
HOOC-CH₂- (D)Cha-Pyr-NH-CH₂-4- (2-am) -thiaz
HOOC-CH₂- (D)Cha-Pyr-NH-CH₂-5- (2-am) -thiaz
HOOC-CH₂- (D)Chg-Pyr-NH-CH₂-2- (5-am) -thiaz
HOOC-CH₂- (D)Chg-Pyr-NH-CH₂-2- (4-am) -thiaz
HOOC-CH₂- (D)Chg-Pyr-NH-CH₂-4- (2-am) - thiaz
HOOC-CH₂- (D)Chg-Pyr-NH-CH₂-5- (2-am) -thiaz
HOOC-CH₂- (D)Cha-Dep-NH-CH₂-2- (5-am) -thiaz
HOOC-CH₂- (D)Cha-Pyr-NH-CH₂-2- (4-am) -oxaz
HOOC-CH₂- (D)Chg-Pyr-NH-CH₂-2- (4-am-) -oxaz
HOOC-CH₂- (D)Cha-Pyr-NH-CH₂-5- (3-am-1-Me) -pyraz
HOOC-CH₂- (D)Cha-Pyr-NH-CH₂-3- (5-am) -1,2,4-oxadiaz
HOOC-CH₂- (D)Cha-Pyr-NH-CH₂-5- (3-am) -1,2,4-oxadiaz
HOOC-CH₂- (D)Chg-Pyr-NH-CH₂-5- (3-am) -1,2,4-oxadiaz
HOOC-CH₂- (D)Cha-Pyr-NH-CH₂-5- (3-am-1-Me) -1,2, 4-triaz
HOOC-CH₂-CH₂- (D)Cha-Pyr-NH-CH₂-5- (2-am) -thioph
HOOC-CH₂-CH₂- (D)Cha-Pyr-NH-CH₂-5- (2-am) -fur
HOOC-CH₂-CH₂- (D)Cha-Pyr-NH-CH₂-5- (1-Me-2-am) -pyrr
(HOOC-CH₂)₂- (D)Cha-Pyr-NH-CH₂-5- (2-am) -thioph
(HOOC-CH₂)₂CH- (D)Cha-Pyr-NH-CH₂-5- (2-am) -thioph
HOOC-CH₂- (D)Chg-Pyr-NH-CH₂-5- (2-am) -1,3,4-thiadaz
HOOC-CH₂- (D)Cha-Pyr-NH-CH₂-5- (2-am) -1,3,4-thiadaz
HOOC-CH₂- (D)Chg-Pyr-NH-CH₂-5- (2-am) -1,3,4-oxadiaz
HOOC-CH₂- (D)Cha-Pyr-NH-CH₂-5- (2-am) -1,3,4-oxadiaz

Abkürzungsliste:
- Adaala:: Adamantylalanin
- Adagly:: Adamantylglycin
- AIBN:: Azobisisobutyronitril
- Ac:: Acetyl
- Ala:: Alanin
- am:: Amidino
- Asp:: Asparaginsäure
- Aze:: Azetidincarbonsäure
- Bn:: Benzyl
- Boc:: tert.Butyloxycarbonyl
- Bu:: Butyl
- Cbz:: Benzyloxycarbonyl
- Cha:: Cyclohexylalanin
- Chea:: Cycloheptylalanin
- Cheg:: Cycloheptylglycin
- Chg:: Cyclohexylglycin
- Cog:: Cyclooctylglycin
- Cpa:: Cyclopentylalanin
- Cpg:: Cyclopentylglycin
- DC:: Dünnschichtchromatographie
- DCC:: Dicyclohexylcarbodiimid
- Dch:: Dicyclohexylalanin
- Dcha:: Dicyclohexylamin
- DCM:: Dichlormethan
- Dep:: 4,5-Dehydropipecolinsäure
- DMF:: Dimethylformamid
- DIPEA:: Diisopropylethylamin
- Dpa:: Diphenylalanin
- Diphe:: 2,5-Dihydrophenylalanin
- Et:: Ethyl
- Eq:: Äquivalente
- Gly:: Glycin
- fur:: Furan
- ham:: Hydroxyamidino
- HOSucc:: Hydroxysuccinimid
- HPLC:: Hochleistungsflüssigkeitschromatographie
- Hyp:: Hydroxyprolin
- imi:: Imidazol
- 2-Ind:: 2-Dihydroindolcarbonsäure
- iPr:: iso-Propyl
- Leu:: Leucin
- Lsg:: Lösung
- Me:: Methyl
- α-MeCha:: α-Methylcyclohexylalanin
- ββ-Me₂Cha:: 2-Amino-3-cyclohexyl-3-methyl-buttersäure oder ββ-Dimethylcyclohexylalanin
- 4-MeCha:: (4-Methylcyclohex-1-yl)alanin
- γ-MeCha:: (1-Methylcyclohex-1-yl)alanin
- 3,3-Me₂Cha:: (3,3-Dimethylcyclohex-1-yl)alanin
- 4-MeChg:: (4-Methylcyhex-1-yl)glycin
- 3,3-Me₂Chg:: (3,3-Dimethylcyclohex-1-yl)-glycin
- MPLC:: Mitteldruckflüssigkeitschromatographie
- MTBE:: Methyl-tert.-butyl-ether
- NBS:: N-Bromsuccinimid
- Nog:: Norbornylglycin
- Oxadiaz:: 1,2,4-Oxadiazol
- Oxaz:: Oxazol
- Ph:: Phenyl
- Phe:: Phenylalanin
- 2Phi:: 2-Perhydroindolcarbonsäure
- Pic:: Pipecolinsäure
- pico:: picolyl
- pim:: piperidinylmethyl
- PPA:: Propylphosphonsäureanhydrid
- Pro:: Prolin
- Py:: Pyridin
- Pyr:: 3,4-Dehydroprolin
- pyraz:: Pyrazol
- pyrr:: Pyrrol
- RT:: Raumtemperatur
- RP-18: Reversed Phase C-18
- t:: tertiär
- tBu:: tertiär-Butyl
- tert:: tertiär
- TBAB:: Tetrabutylammoniumbromid
- TEA:: Trietylamin
- TFA:: Trifluoressigsäure
- TFFA:: Trifluoressigsäureanhydrid
- thiaz:: Thiazol
- thioph:: Thiophen
- 1Tic:: 1-Tetrahydroisochinolincarbonsäure
- 3Tic:: 3-Tetrahydroisochinolincarbonsäure
- TOTU:: O-(Cyan-ethoxycarbonylmethylen)-amino-1-N,N,N',N'-tetramethyluroniumtetrafluoroborat
- triaz:: 1,3,4-Triazol
- Z:: Benzyloxycarbonyl

Gegenstand der Erfindung sind weiter Verbindungen, die das Strukturelement 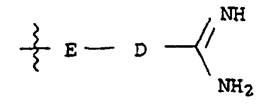 enthalten, worin E und D die oben angegebene Bedeutung besitzen und sich am Stickstoffatom von Baustein E ein Wasserstoffatom, eine Schutzgruppe, eine gegebenenfalls substituierte natürliche oder unnatürliche Aminosäure, eine gegebenenfalls substituierte Carbonsäure oder Sulfonsäure oder ein gegebenenfalls substituierter Alkylrest befindet. Das Strukturfragment ist als Bestandteil von Serinprotease-Inhibitoren und insbesondere von Thrombin- und Kallikreininhibitoren wertvoll.

Gegenstand der Erfindung sind weiter die Zwischenprodukte der Formel IIa und IIb

A ― B ― E ― D ― CN IIa,

A ― B ― E ― D ― CSNH₂ IIb,

worin A, B, E und D die in Anspruch 1 angegebene Bedeutung besitzen.

Die neuen Zwischenprodukte dienen zur Herstellung der Verbindungen I und sind wertvolle Bausteine für die Synthese von Serinprotease-Inhibitoren.

Die Verbindungen der Formel I können als solche oder in Form ihrer Salze mit physiologisch verträglichen Säuren vorliegen. Beispiele für solche Säuren sind: Salzsäure, Zitronensäure, Weinsäure, Milchsäure, Phosphorsäure, Methansulfonsäure, Essigsäure, Ameisensäure, Maleinsäure, Fumarsäure, Bernsteinsäure, Hydroxybernsteinsäure, Schwefelsäure, Glutarsäure, Asparaginsäure, Brenztraubensäure, Benzoesäure, Glucuronsäure, Oxalsäure, Ascorbinsäure und Acetylglycin.

Die neuen Verbindungen der Formel I lassen sich bei folgenden Indikationen einsetzen:
- Krankheiten, deren Pathomechanismus direkt oder indirekt auf der proteolytischen Wirkung von Thrombin beruht,
- Krankheiten, deren Pathomechanismus auf der thrombinabhängigen Aktivierung von Rezeptoren und Signal transduktionen beruht,
- Krankheiten, die mit Stimulation [z.B. durch PAI-1, PDGF (platelet derived growth factor), P-Selectin, ICAM-1, Tissue Factor] oder Inhibition (z.B. NO-Synthese in Glattmuskelzellen) von Genexpressionen in Körperzellen einhergehen,
- Krankheiten, die auf der mitogenen Wirkung von Thrombin beruhen,
- Krankheiten, die auf einer thrombinabhängigen Kontraktilitäts- und Permeabilitätsveränderung von Epithelzellen (z.B. Gefäßendothelzellen) beruhen,
- thrombinabhängige, thromboembolische Ereignisse wie tiefe Venenthrombose, Lungenembolie, Myocard- oder Cerebralinfarkt, Vorhofflimmern, Bypassverschluß,
- disseminierte intravasale Koagulation (DIC),
- Reokklusion und zur Verkürzung der Reperfusionszeit bei Komedikation mit Thrombolytika wie Streptokinase, Urokinase, Prourokinase, t-PA, APSAC, Plasminogenaktivatoren aus den Speicheldrüsen von Tieren sowie die rekombinanten und mutierten Formen all dieser Substanzen,
- das Auftreten von früher Reokklusion und später Restenosierung nach PTCA,
- die thrombinabhängige Proliferation von Glattmuskelzellen,
- die Akkumulation aktiven Thrombins im ZNS(z.B. bei M. Alzheimer),
- das Tumorwachstum sowie gegen die Adhäsion und Metastasierung von Tumorzellen.

Insbesondere lassen sich die neuen Verbindungen zur Therapie und Prophylaxe von thrombinabhängigen thromboembolischen Ereignissen wie tiefen Venenthrombosen, Lungenembolien, Myocard- oder Cerebralinfarkten und instabiler Angina, weiterhin zur Therapie der Disseminierten Intravasalen Koagulation (DIC) einsetzen. Weiter eignen sie sich zur Kombinationstherapie mit Thrombolytika wie Streptokinase, Urokinase, Prourokinase, t-PA, APSAC und anderen Plasminogenaktivatoren zur Verkürzung der Reperfusionszeit und Verlängerung der Reokklusionszeit.

Weitere bevorzugte Anwendungsgebiete sind die Verhinderung thrombinabhängiger früher Reokklusion und später Restenosierung nach perkutaner transluminaler koronarer Angioplasie, die Verhinderung thrombininduzierter Proliferation glatter Muskelzellen, die Verhinderung der Akkumulation aktiven Thrombins im ZNS (z.B. bei M. Alzheimer), die Tumorbekämpfung und die Verhinderung von Mechanismen, die zu Adhäsion und Metastasierung von Tumorzellen führen.

Die neuen Verbindungen lassen sich auch zur Beschichtung von künstlichen Oberflächen wie Hämodialysemembranen und den dazu erforderlichen Schlauchsystemen und Leitungen sowie von Oxygenatoren der extravasalen Zirkulation, Stents und Herzklappen verwenden.

Die neuen Verbindungen lassen sich weiter bei Krankheiten einsezen, deren Pathomechanismus direkt oder indirekt auf der proteolytischen Wirkung von Kininogenasen, insbesondere Kallikrein beruht z.B. bei Entzündungskrankheiten wie Asthma, Pankreatitis, Rhinitis, Arthritis, Urticaria und anderen inneren Entzündungskrankheiten.

Die erfindungsgemäßen Verbindungen können in üblicher Weise oral oder parenteral (subkutan, intravenös, intramuskulär, intraperitoneal, rektal) verabfolgt werden. Die Applikation kann auch mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis pro Person zwischen etwa 10 und 2000 mg bei oraler Gabe und zwischen etwa 1 und 200 mg bei parenteraler Gabe. Diese Dosis kann in 2 bis 4 Einzeldosen oder einmalig am Tag als Depotform gegeben werden.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al.: Pharmazeutische Technologie, Thieme-Verlag, Stuttgart, 1978). Die so erhaltenen Applikationsformen enthalten den Wirkstoff normalerweise in einer Menge von 0,1 bis 99 Gew.-%.

### Experimenteller Teil

Die Verbindungen der Formel I lassen sich entsprechend Schemata I-III darstellen.

Die Bausteine A, B, E und D werden vorzugsweise vorher separat aufgebaut und in geeignet geschützter Form (siehe Schema I-III) eingesetzt. 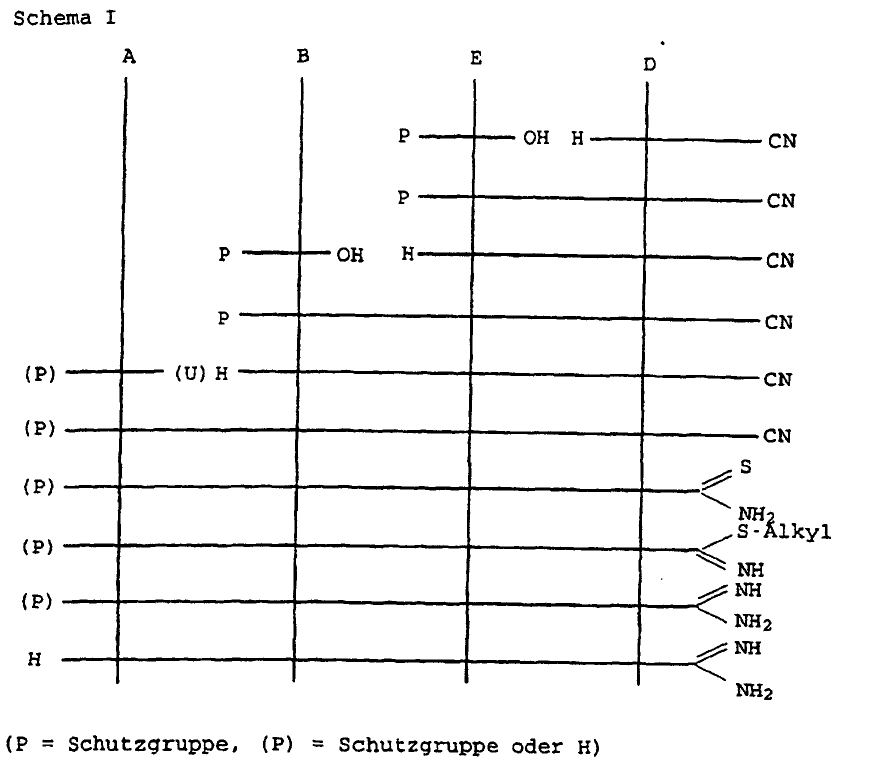 Schema I beschreibt den linearen Aufbau des Moleküls I durch Kupplung des Amins H-D-CN mit der N-geschützten Aminosäure P-E-OH zu P-E-D-CN, Abspaltung der N-terminalen Schutzgruppe zu H-E-D-CN, Kupplung mit der N-geschützten Aminosäure P-B-OH zu P-B-E-D-CN, Abspaltung der Schutzgruppe P zu H-B-E-D-CN, anschließende Alkylierung mit dem gegebenenfalls geschützten (P) -A-U-Baustein (U = Abgangsgruppe) oder reduktive Alkylierung mit (P)-A'-U (U = Aldehyd, Keton) oder Michael-Addition mit einem geeignetem (P)-A"-C=C-Derivat zu (P)-A-B-E-D-CN. Die Umwandlung der Nitrilfunktion in die Amidingruppe erfolgt entweder über die klassische Pinner-Synthese (R. Boder, D.G. Neilson, Chem. Rev. 1962, 61, 179) oder über eine modifizierte Pinner-Synthese, die über Iminothioestersalze als Zwischenstufe abläuft (H. Vieweg et al., Pharmazie 1984, 39, 226) oder direkt nach der Methode von A. Eschenmoser Helv. Chimica Acta 69 (1986) 1224. Anschließend werden im Molekül noch vorhandene Schutzgruppen vorzugsweise durch saure Hydrolyse abgespalten.

Wird der Baustein D als H-D-CONH₂ in der Synthese eingebaut, so erfolgt auf einer der geschützten Zwischenstufen die Dehydratisierung der Amid- zur Nitrilfunktion oder die Umwandlung in die Thioamidfunktionen. Alternativ dazu kann der Baustein D als H-D-CSNH₂ in die Synthese eingesetzt werden.

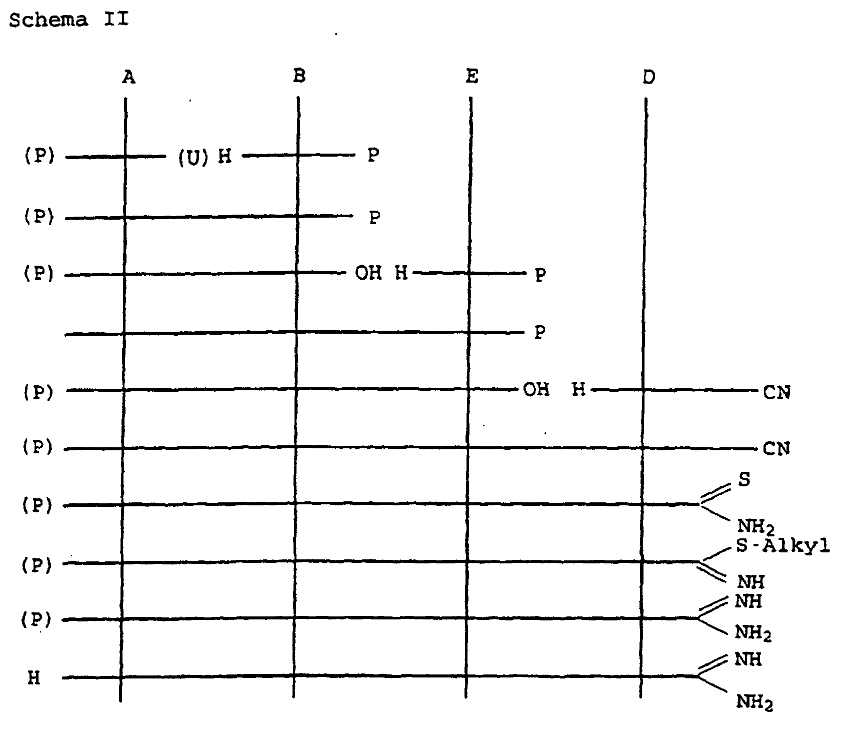

Schema II beschreibt den linearen Aufbau des Moleküls I durch Alkylierung, reduktive Animierung oder Michael-Addition von H-B-P an entsprechend geeignete gegebenenfalls geschützte A-Bausteine zu (P)-A-B-P, Abspaltung der C-terminalen Schutzgruppe zu (P)-A-B-OH, Kupplung ist H-E-P zu (P)-A-B-E-P, Abspaltung der C-terminalen Schutzgruppe zu (P)-A-B-E-OH, Kupplung mit H-D-CN zu (P)-A-E-C-D-CN und Umsetzung dieses Zwischenprodukts zum Endprodukt analog Schema I.

Bei Verbindungen (P)-A-B-P mit noch freier NH-Funktion an B muß diese vor Abspaltung der C-terminalen Schutzgruppe noch mit einer geeigneten Schutzgruppe versehen werden. Die jeweils verwendeten Schutzgruppen müssen orthogonal zueinander sein.

Alternativ zum H-D-CN-Baustein kann auch H-D-CONH₂, H-D-CSNH₂, H-D-C(NH)NH₂, H-D-C(NP)NH₂, H-D-C(NP)NHP eingesetzt werden, wobei im ersten Fall das gekoppelte Zwischenprodukt (P)-A-B-E-D-CONH₂ zu (P)-A-B-E-D-CN dehydratisiert oder direkt in (P)-A-B-E-D-CSNH₂ umgewandelt wird.

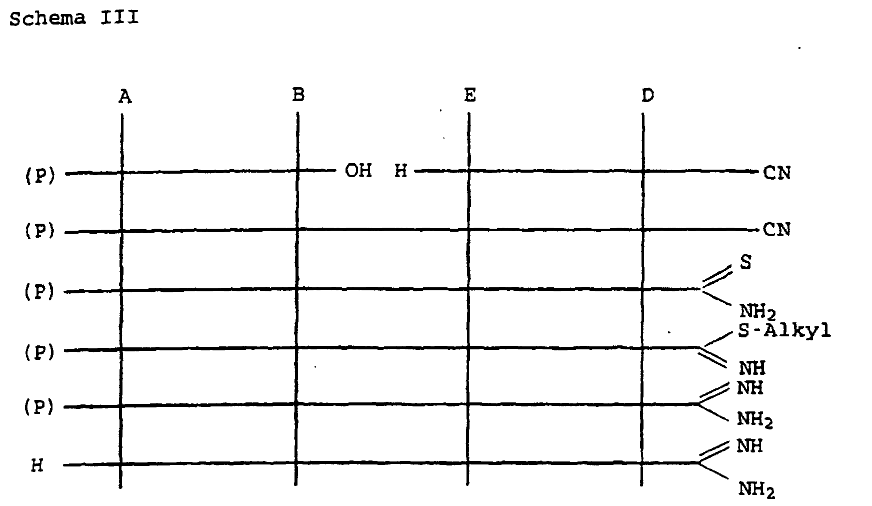

Schema III beschreibt einen sehr effizienten Weg zur Darstellung der Verbindungen I durch eine konvergente Synthese. Die entsprechend geschützten Bausteine (P)-A-B-OH und H-E-D-CN werden miteinander gekuppelt und das entstandene Zwischenprodukt (P)-A-B-E-D-CN analog Schema I zum Endprodukt umgesetzt.

Alternativ zu H-E-D-CN kann auch H-E-D-CONH₂ oder H-E-D-CSNH₂ eingesetzt werden, wobei im ersten Fall das gekoppelte Zwischenprodukt (P)-A-B-E-D-CONH₂ zu (P)-A-B-E-D-CN dehydratisiert oder in (P)-A-B-E-D-CSNH₂ überführt wird.

Als N-terminale Schutzgruppen werden Boc, Cbz oder Fmoc, vorzugsweise Boc eingesetzt, C-terminale Schutzgruppen sind Methyl, tert.-Butyl und Benzyl. Sind mehrere Schutzgruppen im Molekül vorhanden, so müssen diese orthogonal zueinander sein, wenn sie nicht gleichzeitig abgespalten werden sollen. Enthalten die Zwischenprodukte den Baustein E, sind Cbz- und Benzylschutzgruppen ungeeignet.

Die erforderlichen Kupplungsreaktionen sowie die üblichen Reaktionen der Schutzgruppeneinführung und -abspaltung werden nach Standardbedingungen der Peptidchemie durchgeführt (siehe M. Bodanszky, A. Bodanszky "The Practice of Peptide Synthesis", 2. Auflage, Springer Verlag Heidelberg, 1994).

Boc-Schutzgruppen werden mittels Dioxan/HCl oder TFA/DCM, Cbz-Schutzgruppen hydrogenolytisch oder mit HF abgespalten. Die Verseifung von Esterfunktionen erfolgt mit LiOH in einem alkoholischen Lösungsmittel oder in Dioxan/Wasser. t-Butylester werden mit TFA oder Dioxan/HU gespalten.

Die Reaktionen wurden mittels DC kontrolliert, wobei üblicherweise folgende Laufmittel benutzt wurden:

| | | | |
|---|---|---|---|
| A. | DCM/MeOH | | 95:5 |
| B. | DCM/MeOH | | 9:1 |
| C. | DCM/MeOH | | 8:2 |
| D. | DCM/MeOH/50 | %ig HOAc | 40:10:5 |
| E. | DCM/MeOH/50 | %ig HOAc | 35:15:5 |

Sofern säulenchromatographische Trennungen erwähnt werden, waren dies Trennungen über Kieselgel, für die die oben genannten Laufmittel verwendet wurden.

Reversed phase HPLC Trennungen wurden mit Acetonitril/Wasser und HOAc Puffer durchgeführt.

Die Ausgangsverbindungen lassen sich nach folgenden Methoden herstellen:

Als Bausteine A werden für die Alkylierung z.B. α-Bromessigsäuretert.-butylester, β-Brompropionsäure-tert.-butylester, α-Brompropionsäure-tert.-butylester, γ-Brombuttersäure-tert.-butylester, α-Brombuttersäure-tert.-butylester, THP-geschütztes Bromethanol, THP-geschütztes γ-Brompropanol, α-Brom-γ-butyrolacton, für die reduktive Aminierung z.B. Dihydroxyaceton, Acetondicarbonsäureditert.-butylester und für die Michael-Addition z.B. Acrylsäuretert.-butylester, Methacrylsäure-tert.-butylester, Fumarsäure-ditert.-butylester, eingesetzt. Die genannten tert.-Butylester werden, soweit sie nicht käuflich zu erwerben sind, analog G. Uray, W. Lindner, Tetrahedron 1988, 44, 4357-4362 hergestellt.

### B-Bausteine:

Für die allgemeine und spezielle Synthese von Aminosäuren stehen in der Literatur vielfältige Möglichkeiten zur Verfügung. Eine Übersicht hierzu bietet u.a. Band E16d/Teil 1 - Houben-Weyl, S. 406 ff.

Häufig eingesetzte Edukte waren Benzophenoniminessigsäureethylester, Acetamidomalonsäurediethylester und Isonitrilessigsäureethylester.

Die Darstellung verschiedener Glycin-und Alaninderivate erfolgte z.B. ausgehend von Isonitrilessigsäureethylester und einem entsprechenden Keton bzw. Aldehyd (siehe H.-J. Prätorius, J. Flossdorf, M.-R. Kula Chem. Ber. 1975, 108, 3079).

Die Synthesen von Cyclooctylglycin, 2-Norbonylglycin, Adamantylalanin, γ-Methylcyclohexylalanin, 4-Isopropylcyclohex-1-yl-alanin, 4-Methylcyclohex-1-yl-alanin und 4-Methylcyclohex-1-ylglycin wurden über die entsprechenden 2-Formylamino-acrylsäureethylester (U. Schöllkopf und R. Meyer, Liebigs Ann. Chem. 1977, 1174) ausgehend von Isocyanessigsäureethylester mit den jeweiligen Carbonylverbindungen Cyclooctanon, 2-Norbornanon, 1-Formyladamantan, 1-Formyl-1-methyl-cyclohexan, 1-Formyl-4-isopropylcyclohexan, 1-Formyl-4-methyl-cyclohexan und 4-Methylcyclohexanon nach folgenden allgemeinen Vorschriften durchgeführt:

Allgemeine Arbeitsvorschrift zur Synthese der 2-Formylaminoacrylsäureethylester

Zu 100 mMol Kalium-tert.-butylat in 150 ml THF tropfte man bei 0 bis -10°C die Lösung von 100 mMol Isocyanessigsäureethylester in 50 ml THF. Nach 15 min fügte man bei gleicher Temperatur 100 mMol der entsprechenden Carbonylverbindung in 50 ml THF zu, ließ die Reaktionsmischung langsam auf RT ansteigen und zog das Lösungsmittel am Rotationsverdampfer ab. Der Rückstand wurde mit 50 ml Wasser, 100 ml Essigsäure und 100 ml DCM vermischt und das Produkt mit DCM extrahiert. Die DCM-Phase wurde über Na₂SO₄ getrocknet und das Lösungsmittel am Rotationsverdampfer abgezogen. Die fast rein anfallenden Produkte konnten im Bedarfsfall säulenchromatographisch über Kieselgel (Laufmittel: Gemische aus Ether/Petrolether) weiter gereinigt werden.

Allgemeine Vorschrift der Aminosäurehydrochloride ausgehend von den 2-Formylamino-acrylsäureethylestern

100 mMol der 2-Formylamino-acrylsäureethylester wurden mit Pd/C (10 %)-wasserstoff in 200 ml Eisessig bis zur vollständigen Umsetzung hydriert. Dann wurde der Katalysator abfiltriert, die Essigsäure so weit wie möglich am Rotationsverdampfer abgezogen und der Rückstand in 200 ml halbkonzentrierter Salzsäure 5 h zum Rückfluß erhitzt. Man zog die Salzsäure am Rotationsverdampfer ab, trocknete das Produkt bei 50°C im Vakuum und wusch mehrmals mit Ether nach. Die Hydrochloride fielen als schwach gefärbte Kristalle an.

Ausgehend von 18,9 g (150 mMol) Cyclooctanon erhielt man 25,0 g Cyclooctylglycin-hydrochlorid. Ausgehend von 16,5 g (150 mMol) 2-Norbornanon erhielt man 26,6 g 2-Norbonylglycin-hydrochlorid. Ausgehend von 19,7 g (120 mMol) 1-Formyladamantan erhielt man 26,0 g Adamantylalanin-hydrochlorid. Ausgehend von 12,6 g (100 mMol) 1-Formyl-1-methyl-cyclohexan erhielt man 16,6 g γ-Methylcyclohexylalanin-hydrochlorid. Ausgehend von 16,8 g (150 mMol) 4-Methylcyclohexanon erhielt man 25,9 g 4-Methylcyclohexylglycin-hydrochlorid. Ausgehend von 15 g trans-1-Formyl-4-methylcyclohexan erhielt man 18 g trans-4-Methylcyclohex-1-ylalanin-hydrochlorid. Ausgehend von 9 g 3,3-Dimethyl-1-formylcyclohexan erhielt man 10 g 3,3-Dimethylcyclohex-1-yl-alaninhydrochlorid.

Der für die Synthese benötigte Aldehyd, 1-Formyl-3,3-dimethylcyclohexan, wurde in Anlehnung an Moskal und Lensen (Rec. Trav. Chim. Pays-Bas 1987, 106, 137-141) dargestellt:

Eine Lösung von n-Butyllithium in n-Hexan (72 ml, 115 mmol) wurde innerhalb von 10 min bei -60°C zu einer gerührten Lösung von Diethylisocyanomethylphosphonat (17 ml, 105 mmol) in 280 ml wasserfreiem Diethyether getropft. Die entstandene Suspension wurde 15 min bei -60°C nachgerührt und innerhalb von 10 min mit einer Lösung von 3,3-Dimethylcyclohexanon (13 g, 105 mmol) in 100 ml wasserfreiem Diethylether versetzt, wobei die Temepratur unter -45°C gehalten wurde. Man ließ das Reaktionsgemisch auf 0°C kommen, rührte 90 min bei dieser Temperatur und gab vorsichtig 150-200 ml 38 %ige wäßrige Salzsäure hinzu. Zur vollständigen Hydrolyse wurde 15 h lang bei Raumtemperatur heftig gerührt. Man trennte die organische Phase ab, wusch sie mit je 200 ml Wasser, gesättigter Natriumhydrogencarbonat-Lösung und gesättigter Natriumchlorid-Lösung. Man trocknete über Magnesiumsulfat, filtrierte ab und engte am Rotationsverdampfer ein, um die Lösungsmittel zu entfernen. Der erhaltene Rückstand wurde ohne weitere Reinigung als Ausgangsmaterial für die Synthese der Aminosäure eingesetzt.

Boc-(D)-α-methyl-cyclohexylalanin 3,4 g (12,2 mMol) Boc-(D)-α-Methyl-Phe-OH wurden in 100 ml MeOH bei 50°C in Gegenwart von 250 mg 5-%igem Rh auf Al₂O₃ 24 h bei 10 bar mit Wasserstoff hydriert. Man erhielt nach Filtration und Abziehen des Lösungsmittels 2,8 g Boc-(D)-α-Methyl-Cha-OH.
¹H-NMR (DMSO-d⁶, δ in ppm): 12 (sehr breites Signal, COOH); 1.7-0,8 (25 H; 1,35 (s, Boc), 1,30 (s, Me))

Boc-(3-Ph)-Pro-OH wurde analog einer Vorschrift von J.Y.L. Chung et al. (J.Y.L. Chung et al. J.Org.Chem. 1990, 55, 270) synthetisiert.

### Darstellung von Boc-1-Tetralinylglycin

Boc-1-Tetralinylglycin wurde ausgehend von 1,2-Dihydronaphthalin dargestellt. 1,2 Dihydronaphthalin wurde zunächst mit HBr in 1-Tetralylbromid überführt (analog J. Med. Chem 1994, 37, 1586). Anschließend wurde das Bromid mit Acetamidomalonsäurediethylester umgesetzt, hydrolytisch gespalten und die erhaltene α-Aminosäure unter Standardbedingungen in die Boc geschützte Form überführt. Eine weitere Darstellungsmöglichkeit wird von E. Reimann und D. Voss beschrieben (E. Reimann; D. Voss Arch. Pharm 1977, 310, 102).

### Darstellung von Boc-1-(D,L)Tic-OH

Boc-1-(D,L)Tic-OH wurde nach einer Vorschrift von R. T. Shuman et al. dargestellt (R.T. Shuman et al. J. Med. Chem. 1993, 36, 314).

### Darstellung von Boc-(D,L)Dch-OH

Boc-(D,L)-Dpa-OH (1mmol) wurde in 12 ml MeOH zusammen mit katalytischen Mengen von 5 % Rh/Al₂O₃ bei 5 bar hydriert. Nach Filtration und Entfernen des Solvens im Vakuum erhielt man das Produkt in quantitativer Ausbeute.

Darstellung von Cycloheptylglycin, Cyclopentylglycin, 4-Isopropylcyclohexylglycin und 3,3-Dimethylcyclohexylglycin

Die Darstellung dieser Aminosäuren erfolgte durch Umsetzung von Cycloheptanon, Cyclopentanon, 4-Isopropylcyclohexanon bzw. 3,3-Dimethylcyclohexanon mit Isonitrilessigsäureethylester entsprechend einer Vorschrift von H.-J. Prätorius (H.-J. Prätorius, J. Flossdorf, M.Kula, Chem. Ber. 1985, 108, 3079)

### Darstellung von H-D,L-Chea-OH

4,0 g Cycloheptylmethylmethansulfonat (19,39 mMol), hergestellt aus Cycloheptylmethanol und Methansulfonsäurechlorid, wurden zusammen mit 4,9 g Benzophenoniminglycinethylester (18,47 mMol), 8,9 g trockenem fein gepulvertem Kaliumcarbonat (64,65 mMol) und 1 g Tetrabutylammoniumbromid (3 mMol) in 50 ml trockenem Acetonitril 10 h in Inertgasatmosphäre auf Rückfluß erhitzt. Danach wurde das Kaliumcarbonat abfiltriert, das Filtrat zur Trockene eingedampft, und das Rohprodukt direkt mit 20 ml 2N Salzsäure in 40 ml Ethanol 1,5 h unter Rühren bei RT hydrolisiert. Nach Verdünnen der Reaktionslösung wurde mit Essigester im sauren Bereich Benzophenon extrahiert, anschließend im alkalischen Bereich (pH = 9) H-D,L-Chea-OEt mit DCM extrahiert, die Lösung über Magnesiumsulfat getrocknet und einrotiert. Ausbeute 3,7 g =̂ 95 % der Theorie.

Die Darstellung von Boc-(D,L)-(3,4,5-(MeO)₃)Phe-OH erfolgte durch Alkylierung von Benzophenoniminglycinethylester mit Trimethoxybenzylchlorid, anschließender Boc-Schutzgruppeneinführung und Esterverseifung.

Die Darstellung von D-(1,4-Cyclohexadien-1-yl)ala-OH erfolgte nach G. Zivilichovsky, V. Gurvich J. Chem. Soc., Perkin Trans I 19 (1995) 2509-15.

Die Darstellung von H-(D,L)-ββ-Me₂Cha-OH erfolgte nach U. Schöllkopf, R. Meyer, L. Ann. Chem. 1977, 1174-82.

Die genannten Aminosäuren wurden nach allgemein bekannten Verfahren mit Di-tert.-butyl-dicarbonat in Wasser/Dioxan in die jeweils Boc-geschützte Form überführt und anschließend aus Essigester/Hexan-Gemischen umkristallisiert oder säulenchromatographisch über Kieselgel (Laufmittel: Essigester/Petrolether-Gemische) gereinigt.

Die Boc-geschützten Aminosäuren wurden als B-Bausteine entsprechend Schema I eingesetzt.

Die genannten Aminosäuren wurden als B-Bausteine teilweise auch in die entsprechenden Benzylester überführt und mit den entsprechend geschützten A-Bausteinen verknüpft. Bei Verbindungen mit noch freier N-H-Funktion wurde diese anschließend mit einer Boc-Gruppe geschützt, die Benzylestergruppe abhydriert und der Baustein A-B-OH durch Kristallisation, Salzfällung bzw. Säulenchromatographie gereinigt. Dieser Weg ist exemplarisch für tBuOOC-CH₂-(Boc)(D)Cha-OH nachfolgend beschrieben.

### Synthese von D-Cyclohexylalaninbenzylester

Eine Suspension von 100 g (481 mmol) D-Cyclohexylalanin-hydrochlorid, 104 g (962 mmol) Benzylalkohol und 109,7 g (577 mmol) p-Toluolsulfonsäuremonohydrat in 2200 ml Toluol wurde am Wasserabscheider langsam zum Rückfluß erhitzt. In einem Temperaturbereich von 80-90°C beobachtete man Chlorwasserstoffentwicklung sowie das Auflösen der Suspension zu einer klaren Lösung. Als sich kein Wasser mehr abschied (ca. 4 h), destillierte man 500 ml Toluol ab, ließ die Reaktionsmischung über Nacht abkühlen, filtrierte den entstandenen Rückstand ab und wusch zweimal mit je 1000 ml Hexan nach. Der erhaltene Rückstand (195 g) wurde sodann in 2000 ml Dichlormethan aufgeschlämmt, mit 1000 ml Wasser versetzt und unter Rühren durch sukzessive Zugabe von 50 %iger Natronlauge auf pH 9-9,5 eingestellt. Man trennte die organische Phase ab, wusch sie zweimal mit je 500 ml Wasser, trocknete sie über Natriumsulfat, filtrierte vom Trockenmittel ab und engte das Filtrat ein, wodurch man 115 g (94 %) des Titelproduktes als helles Öl erhielt.

### N- (tert. -butyloxycarbonylmethylen) -D-cyclohexylalaninbenzylester

115 g (440 mmol) D-Cyclohexylalaninbenzylester wurden in 2000 ml Acetonitril gelöst, bei Raumtemperatur mit 607,5 g (4,40 mol) Kaliumcarbonat und 94,3 g (484 mmol) Bromessigsäure-tert.-butylester versetzt und 3 Tage bei dieser Temperatur gerührt. Man filtrierte vom Carbonat ab, wusch mit Acetonitril nach, engte die Mutterlauge ein (30°C, 20 mbar), nahm den Rückstand in 1000 ml Methyl-tert.-butylether auf und extrahierte die organische Phase mit 5 %iger Zitronensäure und gesättigter Natriumhydrogencarbonat-Lösung. Man trocknete die organische Phase über Natriumsulfat, filtrierte vom Trockenmittel ab, engte ein und setzte das erhaltene Öl (168 g) direkt in die folgende Reaktion ein.

### N-Boc-N-(tert.-butyloxycarbonylmethylen)-D-cyclohexylalaninbenzylester

Das in voriger Synthese erhaltene Öl (168 g, 447 mmol) wurde in 1400 ml Acetonitril gelöst, mit 618 g (4,47 mol) Kaliumcarbonat-Pulver und 107,3 g (492 mmol) Di-tert.-butyldicarbonat versetzt und 6 Tage bei Raumtemperatur gerührt. Man saugte das Kaliumcarbonat ab, wusch mit ca. 1000 ml Acetonitril nach und engte das Filtrat ein. Man erhielt 230 g des gewünschten Produkts.

### N-Boc-N-(tert.-butyloxycarbonylmethylen)-D-cyclohexylalanincyclohexylammoniumsalz

115 g N-Boc-N-(tert.-butyloxycarbonylmethylen)-D-cyclohexylalaninbenzylester wurden in 1000 ml reinem Ethanol gelöst und bei 25-30°C in Gegenwart von 9 g 10 %igem Pd auf Aktivkohle 2 h bei Normaldruck mit Wasserstoff hydriert. Nach Filtration und Entfernen des Lösungsmittels am Rotationsverdampfer erhielt man 100 g (260 mmol) eines gelben Öls, das man in 1600 ml Aceton aufnahm und zum Rückfluß erhitzte. Man entfernte das Heizbad und gab über einen Tropftrichter zügig eine Lösung von 27 g (273 mmol) Cyclohexylamin in Aceton hinzu. Beim Abkühlen der Reaktionsmischung auf Raumtemperatur kristallisierte das gewünschte Salz aus. Man filtrierte den Feststoff ab, wusch mit 200 ml Aceton nach und kristallisierte zur endgültigen Reinigung noch einmal aus Aceton um. Nach Trocknung des Rückstandes im Vakuumtrockenschrank bei 30°C erhielt man 70,2 g des gewünschten Salzes als weißes Pulver.

N-Boc-N-(tert.-butyloxycarbonylmethylen)-D-cyclohexylglycincyclohexylammoniumsalz wurde in analoger Weise aus Cyclohexylglycin als Edukt hergestellt.

N-Boc-N-(tert.-butyloxycarbonylethylen)-D-cyclohexylalanin-cyclohexylammoniumsalz
a) 3-Brom-propionsäure-tert-butylester
   16,64g (109 mmol) Brompropionsäure, 150 ml kondensiertes 2-Methylpropen und 2ml konzentrierte Schwefelsäure wurden bei - 30°C im Stickstoffgegenstrom in ein für einen Autoklaven geeignetes Glasgefäß gegeben, fest verschlossen und 72 h bei Raumtemperatur gerührt. Zur Aufarbeitung wurde das Reaktionsgefäß erneut auf -30°C abgekühlt und die Reaktionslösung vorsichtig in 200ml einer eiskalten, gesättigten Natriumhydrogencarbonatlösung gegossen. Unter Rühren ließ man überschüssiges 2-Methylpropen abdampfen, extrahierte den Rückstand dreimal mit je 50 ml Dichlormethan, trocknete die vereinigten organischen Phasen über Natriumsulfat, filtrierte vom Trockenmittel ab und engte im Wasserstrahlvakuum ein. Der ölige Rückstand wurde säulenchromatographisch gereinigt (Laufmittel n-Hexan, später n-Hexan/Diethylether 9:1). Man erhielt 18,86g der Titelverbindung.
b) N-(tert.-butyloxycarbonylethylen)-D-cyclohexylalaninbenzylester
   49,4 g (189 mmol) D-Cyclohexylalaninbenzylester wurden in 250 ml Acetonitril gelöst, bei Raumtemperatur mit 31.6 g (151 mmol) Brompropionsäure-tert.-butylester versetzt und 5 Tage unter Rückfluß gekocht. Man filtrierte vom entstandenen Niederschlag ab, wusch mehrmals mit Acetonitril nach, engte das Filtrat im Wasserstrahlvakuum ein, nahm den Rückstand in 350ml Dichlormethan auf und extrahierte die organische Phase mit 5 %iger Zitronensäure und gesättigter Natriumhydrogencarbonat-Lösung. Man trocknete die organische Phase über Natriumsulfat, filtrierte vom Trockenmittel ab und engte ein. Der ölige Rückstand wurde säulenchromatographisch gereinigt (Laufmittel Dichlormethan, später Dichlormethan/ Methanol 95:5). Man erhielt ein leicht verunreinigtes Öl, das direkt in die folgende Reaktion eingesetzt wurde.
c) N-Boc-N-(tert.-butyloxycarbonylethylen)-D-cyclohexylalaninbenzylester
   Das in voriger Synthese erhaltene Öl (30g, max. 70mmol) wurde in 150 ml Acetonitril gelöst, mit 28ml (160 mmol) Di-isopropylethylamin und 19,2 g (88 mmol) Di-tert.-butyldicarbonat versetzt und 3 Tage bei Raumtemperatur gerührt. Man engte das Reaktionsgemisch am Rotationsverdampfer im Wasserstrahlvakuum ein, nahm den Rückstand in n-Hexan auf und wusch fünfmal mit je 3ml einer 5%igen Zitronensäurelösung, trocknete die vereinigten organischen Phasen über Natriumsulfat, filtrierte das Tockenmittel ab, engte ein und unterwarf den Rückstand einer säulenchromatographischen Trennung (Laufmittel Hexan/Essigsäureethylester 95:5). Man erhielt 32,66 g (64 mmol) des gewünschten Produkts.
d) N-Boc-N-(tert.-butyloxycarbonylethylen)-D-cyclohexylalanincyclohexylammoniumsalz
   32,66 g (64 mmol) N-Boc-N-(tert.-butyloxycarbonylethylen)-Dcyclohexylalaninbenzylester wurden in 325 ml reinem Ethanol gelöst und bei 25-30°C in Gegenwart von 3 g 10 %igem Pd auf Aktivkohle 14 h bei Normaldruck mit Wasserstoff hydriert. Nach Filtration der Lösung über Celite®, Nachwaschen mit Ethanol und Entfernen des Lösungsmittels am Rotationsverdampfer erhielt man 26,7 g eines gelben Öls, das man in Aceton aufnahm und zum Rückfluß erhitzte. Man entfernte das Heizbad und gab über einen Tropftrichter zügig eine Lösung von 7 g (70 mmol) Cyclohexylamin in Aceton hinzu. Beim Abkühlen der Reaktionsmischung auf Raumtemperatur kristallisierte das gewünschte Salz aus. Man filtrierte den Feststoff ab, wusch mit 25 ml Aceton nach und kristallisierte zur endgültigen Reinigung noch einmal aus Aceton um. Nach Trocknung des Rückstandes im Vakuumtrockenschrank bei 30°C erhielt man 26,6 g (54 mmol) des gewünschten Salzes als weißes Pulver.

N-Boc-N-(tert. butyloxycarbonylmethylen)-(D)-cyclohexylalanyl-3,4-dehydroprolin:
a) N-Boc-Pyr-OH (5 g, 23,45 mmol) wurde in MeOH (50 ml) gelöst und mit HCl in Dioxan (4N, 30 ml) versetzt. Anschließend wurde 12 h unter Rückfluß erwärmt. Das Lösungsmittel wurde abrotiert und H-Pyr-OMe Hydrochlorid als Produkt erhalten. Ausbeute: 3,84 g (100%).
b) N-(t-BuO₂C-CH₂)-N-Boc-(D)-Cha-OH (8 g, 20.75 mmol) wurde in Dichlormethan (75 ml) gelöst und bei -10°C mit Ethyldiisopropylamin (15,5 ml, 89,24 mmol) versetzt. Nach 5 min Rühren bei dieser Temperatur wurde eine Lösung von H-Pyr-OMe Hydrochlorid (3,4 g, 20,75 mmol) in Dichlormethan (25 ml) zugetropft. Anschließend wurde eine Lösung von Propanphosphonsäureanhydrid in Essigsäureethylester (50%ig, 20 ml, 26,96 mmol) zugetropft und 2 h bei -10 bis 0°C gerührt.Der Ansatz wurde mit Dichlormethan verdünnt und mit gesättigter Natriumhydrogencarbonatlösung (2 x 80 ml), 5%iger Zitronensäurelösung (2 x 15 ml) und gesättigter Natriumchloridlösung (1 x 20 ml) gewaschen. Die organische Phase wurde über Natriumsulfat getrocknet und das Lösungsmittel abrotiert. Das Rohprodukt wurde mittels Flashchromatographie gereinigt (Kieselgel, Dichlormethan/Methanol = 95/5). Ausbeute: 6,2 g (60 %).
c) N-(t-BuO₂C-CH₂)-N-Boc-(D)-Cha-Pyr-OMe (5,5 g, 11,12 mmol) wurde in Dioxan (40 ml) gelöst, mit Natronlauge (1N, 22,2 ml, 22,24 mmol) versetzt und 2 h bei Raumtemperatur gerührt. Das Dioxan wurde abrotiert, die wässrige Phase mit Essigsäureethylester gewaschen und mit Kaliumhydrogensulfatlösung (20%ig) auf pH 1-2 angesäuert. Die wässrige Phase wurde mit Dichlormethan extrahiert und die vereinigten organischen Phasen über Natriumsulfat getrocknet. Ausbeute: 5 g (94%), farbloser Schaum. Umkristallisation aus mit Wasser gesättigtem n-Hexan ergab farblose Kristalle (m.p. = 158-160°C).

### N-Boc-N- (tert.-butyloxycarbonylmethylen)-(D)-cyclohexylglycyl-3,4-dehydroprolin

Diese Verbindung wurde in analoger Weise aus N-Boc-N-(tert.butyloxycarbonylmethylen)-(D)-cyclohexylglycin und 3,4-Dehydroprolinmethylester dargestellt.

Das als E-Baustein eingesetzte (L) 3,4-Dehydroprolin ist käuflich zu erwerben, die (D,L)-4,5-Dehydropipecolinsäure läßt sich nach A. Burgstahler, C.E. Aiman J. Org. Chem. 25 (1960), 489 oder C. Herdeis, W. Engel Arch. Pharm 326 (1993), 297 herstellen und anschließend mit (Boc)₂O in Boc-(D,L)-Dep-OH überführen.

Die Synthese der D-Bausteine wurde wie folgt durchgeführt:

5-Aminomethyl-2-cyanothiophen

Die Darstellung dieses Bausteins wurde wie in WO 95/23609 beschrieben, durchgeführt

4-Aminomethyl-2-cyanothiophen
a) 2-Brom-4-formylthiophen
   36 g (320 mmol) 3-Formylthiophen wurden in 600 ml Methylenchlorid gelöst, auf 5°C abgekühlt, portionsweise mit 100 g (750 mmol) Aluminiumtrichlorid versetzt und das Reaktionsgemisch anschließend unter Rückfluß erhitzt. Innerhalb von 45 min tropfte man eine Lösung von 59 g (19 ml, 360 mmol) Brom in 40 ml Methylenchlorid zu und ließ 4 h bei Rückfluß nachreagieren. Nach Abkühlen wurde die Reaktionslösung auf 600 g Eiswasser gegossen, mit Methylenchlorid extrahiert, die organische Phase mit gesättigter Natriumhydrogencarbonatlösung gewaschen, über Magnesiumsulfat getrocknet und im Vakuum einrotiert. Man erhielt 64,5 g Rohprodukt, welches mittels Säulenchromatographie (Kieselgel, Methylenchlorid/Petrolether) gereinigt wurde. Dabei wurden insgesamt 56,5 g leicht verunreinigtes Produkt erhalten.
b) 2-Cyano,-4-formylthiophen
   Zu einer Lösung von 13,53 g (70,82 mmol) 2-Brom-4-formylthiophen in 25 ml DMF wurden 7,6 g (85 mmol) Kupfer(I)cyanid zugegeben und die Reaktionsmischung 3,5 h unter Rückfluß erhitzt, wobei sich die ursprünglich hellgrüne Suspension in eine schwarze Lösung verwandelte. Nach Zugabe von Wasser wurde die Reaktionsmischung mit Essigester mehrfach extrahiert, die organischen Phasen vereinigt, mit gesättigter Kochsalzlösung gewaschen, über Natriumsulfat getrocknet und unter leichtem Vakuum einrotiert. Durch Versetzen des Rückstandes (7 g) mit Ether konnten 1,6 g reines Produkt erhalten werden. Die Mutterlauge wurde zusammen mit den Rohprodukten aus anderen Ansätzen chromatographisch gereinigt. (Kieselgel, Methylenchlorid/Petrolether 1:1). Insgesamt wurden 56,5 g 2-Brom-4-formylthiophen zu 2-Cyano-4-formylthiophen umgesetzt und dabei 12,6 g reines Produkt erhalten (31 % Ausbeute).
c) 2-Cyano-3-hydroxymethylthiophen
   Zu einer Suspension von 12,6 g (91,8 mmol) 2-Cyano-4-formylthiophen in 200 ml Ethanol wurden 3,47 g (91,8 mmol) Natriumborhydrid portionsweise zugegeben und bei Reumtemperatur 2 h gerührt, wobei die Reaktionsmischung langsam eine klare Lösung bildete. Nach Einengen im Vakuum wurde der Rückstand in Essigester aufgenommen, nacheinander mit gesättigter Kochsalzlösung, 5 %iger Zitronensäure und gesättigter Kochsalzlösung gewaschen, die organische Phase mit Natriumsulfat getrocknet und im Vakuum eingeengt. Man erhielt 11,7 g fast reines Produkt (Ausbeute 91,5 %).
d) 3-Brommethyl-2-cyanothiophen
   11,7 g (84,07 mmol) 2-Cyano-3-hydroxymethylthiophen wurden zusammen mit 24,1 g (91,87 mmol) Triphenylphosphin in 100 ml THF bei Raumtemperatur gelöst und unter Kühlung (Eisbad) portionsweise mit 30,47 g (91,87 mmol) Tetrabrommethan versetzt. Nach 3-stündigem Rühren bei Raumtemperatur wurde im Vakuum eingeengt und über Kieselgel (Methylenchlorid/Petrolether) chromatographisch gereinigt. Man erhielt 18,8 g noch Petrolether enthaltendes kristallines hellgelbes Produkt.
e) 4-N,N-Bis(tert-butoxycarbonyl)-aminomethyl-2-cyanothiophen
   18,81 g 3-Brommethyl-2-cyanothiophen (Rohprodukt, maximal 84,07 mmol) wurden in 160 ml THF gelöst, auf 5°C abgekühlt und portionsweise mit 3,07 g (102,4 mmol) 80%iger Natriumhydridsuspension versetzt. Anschließend wurden 22,25 g (102,4 mmol) Di-tert.-butyl-iminodicarboxylat gelöst in 160 ml THF bei 5°C zugetropft und danach über Nacht bei Raumtemperatur gerührt. Da laut DC der Umsatz unvollständig war, wurde 4,5 h auf 30-35°C erwärmt. Nach Abkühlen auf 0-5°C wurden langsam 33 ml gesättigte Ammoniumchloridlösung zugetropft, THF im Vakuum abdestilliert, der Rückstand mehrfach mit Essigester extrahiert, die Essigesterphasen mit gesättigter Kochsalzlösung gewaschen, über Natriumsulfat getrocknet und im Vakuum einrotiert. Der rote zähflüssige Rückstand (34,61 g) wurde als Rohprodukt in der nachfolgenden Umsetzung eingesetzt.
f) 4-Aminomethyl-2-cyanothiophen-Hydrochlorid
   34,61 g 4-N,N-Bis(tert.-butoxycarbonyl)-aminomethyl-2-cyanothiophen (Rohprodukt, maximal 84,07 mmol) wurden in 600 ml Essigester gelöst, auf 0-5°C abgekühlt, mit HCl-Gas gesättigt und auf Raumtemperatur erwärmt. Nach 3 h rotierte man die entstandene Suspension ein, kodestillierte mehrfach mit Methylenchlorid, rührte den Rückstand mit Ether aus und trocknete den Rückstand im Vakuum. Es wurden 13,85 g Produkt als helles Pulver erhalten. Ausbeute über zwei Stufen 94,3 %.

### 2-Aminomethyl-4-cyanothiophen

a) 4-Cyano-thiophen-2-carbaldehyd
   49,3 g (258,05 mmol) 4-Brom-thiophen-2-carbaldehyd und 27,8 g (310,41 mmol) Kupfer-(I)-cyanid wurden in 130 ml absolutem DMF suspendiert und 8 h unter Rückfluß erhitzt. Das Lösungsmittel wurde im Vakuum bei 40°C abrotiert, der Rückstand in Essigester suspendiert und in eine Soxleth-Apparatur überführt. Der Rückstand wurde über Nacht extrahiert, die gelbe Lösung über Natriumsulfat getrocknet, im Vakuum einrotiert und der erhaltene gelbe Feststoff aus Ether umkristallisiert. Es wurden 25,3 g Produkt erhalten (80 % der Theorie)
b) 4-Cyano-thiophen-2-carbaldehyd-oxim
   11,6 g (84,6 mmol) 4-Cyano-thiophen-2-carbaldehyd wurden in 140 ml Methanol gelöst und mit 12,3 g (116,1 mmol) Natriumcarbonat versetzt. Anschließend wurden 6,5 (93,5 mmol) Hydroxylamin-Hydrochlorid portionsweise unter Kühlung bei 15°C zugegeben und noch 2 h bei 10°C gerührt. Nach Zugabe von 80 ml Wasser extrahierte man die Reaktionsmischung fünfmal mit je 50 ml Diethylether, trocknete die organische Phase über Natriumsulfat und entfernte das Lösungsmittel im Vakuum. Es wurden 12,5 g gewünschtes Produkt als gelbes Kristallpulver erhalten (96 % der Theorie)
c) 2-Aminomethyl-4-cyanothiophen-Hydrochlorid
   11,22 g (171,64 mmol) feiner Zinkstaub wurden in mehreren kleinen Portionen vorsichtig zu einer auf 0-5°C gekühlten Lösung von 4,65 g (30,60 mmol) 4-Cyano-thiophen-2-carbaldehyd-oxim in 50 ml Trifluoressigsäure so zugegeben, daß die Temperatur 15°C nicht überstieg. Nach 3 h Rühren bei Raumtemperatur wurde vom überschüssigen Zink abdekandiert, Trifluoressigsäure im Vakuum (Ölpumpe) weitgehend entfernt, das verbliebene Öl auf 0°C abgekühlt und portionsweise mit einer auf 0°C vorgekühlten Mischung aus 150 ml 3N Natronlauge und 2 1 Methylenchlorid versetzt. Nach Filtration von Unlöslichem wurde die organische Phase abgetrennt, die wäßrige Phase achtmal mit 20 ml Methylenchlorid extrahiert, die gesammelten organischen Phasen über Natriumsulfat getrocknet und anschließend unter Eiskühlung mit 20 ml 6M methanolischer Salzsäure versetzt. Dabei fiel das Produkt in Form des Hydrochlorids als weißer Feststoff aus, wobei zur Vervollständigung der Kristallisation die Suspension über Nacht auf 4°C abgekühlt wurde. Es wurden 2,2 g Produkt als farblose Nadeln erhalten (50 % der Theorie)

5-Aminomethyl-3,4-dimethyl-thiophen-2-carbonsäureamid-Hydrochlorid

19 g (105,42 mmol) 5-Cyano-3,4-dimethyl-thiophen-2-carbonsäureamid wurden in 760 ml Methanol und 110 ml 2N Salzsäurelösung suspendiert, mit 9,5 g Pd auf Kohle (10%) versetzt und bei Raumtemperatur hydriert. Nach Aufnahme von 4,7 1 Wasserstoff (4 h) wurde Methanol im Vakuum abdestilliert, die Wasserphase dreimal mit Essigester extrahiert und die wäßrige Phase anschließend gefriergetrocknet. Man erhielt 16,3 g gewünschtes Produkt als weiße Festsubstanz (70,4 % der Theorie).

### 5-Aminomethyl-isoxazol-3-carbonsäureamid

a) 5-Chlormethyl-isoxazol-3-carbonsäureethylester
   Zu einer auf 10-15°C abgekühlten Mischung aus 30 g (198 mmol) 2-Chlor-2-hydroxyimino-essigsäureethylester und 150 ml Propargylchlorid wurden 21,2 g (210 mmol) Triethylamin unter Rühren zugetropft, 1 h bei Raumtemperatur nachgerührt, anschließend mit Wasser versetzt, mit Ether extrahiert, die organische Phase über Magnesiumsulfat getrocknet und im Vakuum einrotiert. Der Rückstand wurde im Vakuum bei 0,5 Torr destilliert, wobei das Produkt bei 116-122°C überdestillierte.
b) 5-Chlormethyl-isoxazol-3-carbonsäure
   47,3 g (250 mmol) 5-Chlormethyl-isoxazol-3-carbonsäureethylester wurden in 150 ml Ethanol mit 14 g (250 mmol) Kaliumhydroxid versetzt und die Reaktionsmischung 6 h bei 60-70°C gerührt. Nach dem Abkühlen wurde im Vakuum eingeengt, der Rückstand in Wasser aufgenommen, mit Ether extrahiert, die wäßrige Phase mit Salzsäure angesäuert, anschließend mehrfach mit Ether extrahiert, die Etherphase über Natriumsulfat getrocknet und im Vakuum eingeengt (Ölpumpe, 50°C). Es wurden 31 g des gewünschten Produktes erhalten (77 % der Theorie)
c) 5-Chlormethyl-isoxazol-3-carbonsäurechlorid
   120 g (743 mmol) 5-Chlormethyl-isoxazol-3-carbonsäure wurden zusammen mit 500 ml Thionylchlorid und 2 Tropfen Pyridin 10 h unter Rückfluß erhitzt, anschließend im Vakuum eingeengt und dann bei 20 Torr destilliert. Das Produkt destillierte bei 125-133°C. Es wurden 78 g erhalten (58 % der Theorie)
d) 5-Chlormethyl-isoxazol-3-carbonsäureamid
   In eine Lösung von 10 g (55,56 mmol) 5-Chlormethyl-isoxazol-3-carbonsäurechlorid in 100 ml Methylenchlorid wurde bei 10-15°C 1 h Ammoniak eingeleitet und anschließend 1 h bei Raumtemperatur weitergerührt. Nach Abkühlen der Lösung auf 0°C wurde der Niederschlag abgesaugt, mit wenig kaltem Methylenchlorid gewaschen und der Rückstand 2mal mit Wasser zur Entfernung der Ammoniumsalze ausgerührt. Nach dem Trocknen im Vakuum wurden 6,58 g reines Produkt als helles Pulver erhalten (74 % der Theorie)
e) 5-Aminomethyl-isoxazol-3-carbonsäureamid-Hydrochlorid
   Zu einer Mischung aus 100 ml konzentrierter Ammoniaklösung und 72 ml Methanol wurden 2,44 g (15,2 mmol) 5-Chlormethylisoxazol-3-carbonsäureamid gegeben, die Reaktionslösung auf 40°C erwärmt und dabei ständig mit Ammoniakgas gesättigt. Nach 6 h war das Edukt umgesetzt. Das Methanol wurde im Vakuum entfernt, die wäßrige Phase zweifach mit Methylenchlorid extrahiert und anschließend die wäßrige Phase schonend im Vakuum zur Trockene einrotiert. Der weiße feste Rückstand wurde als Rohprodukt in die Kupplung mit Boc-Dehydroprolin eingesetzt.

2-Aminomethyl-thiazol-4-thiocarboxamid wurde entsprechend G. Videnov, D. Kaier, C. Kempter und G. Jung Angew. Chemie (1996) 108, 1604 dargestellt, wobei die dort beschriebene N-Boc-geschützte Verbindung mit etherischer Salzsäure in Methylenchlorid entschützt wurde.

### 4-Aminomethyl-thiazol-2-thiocarboxamid

Die Vorstufe 4-Aminomethyl-thiazol-2-carbonsäureethylester wurde entsprechend dem Patent US 4 826 816 hergestellt. Nach Einführung der Boc-Schutzgruppe an der Aminfunktion wurde die Estergruppe verseift, die entstandene Säurefunktion über das gemischte Anhydrid (Kohlensäureisobutylester) in das Carbonsäureamid und anschließend mit Lawesson Reagenz in das Thioamid überführt. Nach Schutzgruppenabspaltung wurde die oben angegebene Zwischenverbindung erhalten.

### 5-Aminomethyl-2-cyanofuran

a) 5-Cyanofuran-2-carbaldehyd:
   Zu einer auf -78°C gekühlten Lösung von 26,7 g (264 mmol) Diisopropylamin in 600 ml Tetrahydrofuran gab man innerhalb von 20 min 165 ml (264 mmol) einer 1,6 molaren Lösung von n-Butyllithium in n-Hexan. Die Lösung ließ man auf -20°C kommen, kühlte erneut auf -75°C und tropfte bei dieser Temperatur langsam eine Lösung von 22,3 g (240 mmol) 2-Cyanofuran in 100 ml Tetrahydrofuran hinzu. Man ließ 30 min nachrühren, tropfte langsam 93 ml Dimethylformamid hinzu und ließ erneut 30 min rühren. Zur Aufarbeitung versetzte man bei - 70°C mit einer Lösung von 40 g Zitronensäure in 200 ml Wasser. Man engte am Rotationsverdampfer ein, versetzte mit 600 ml gesättigter Natriumchloridlösung und extrahierte dreimal mit je 200 ml Diethylether. Die vereinigten organischen Extrakte wurden über Magnesiumsulfat getrocknet. Nach dem Abfiltrieren des Trockenmittels wurde das Lösungsmittel im Wasserstrahlvakuum abdestilliert und der Rückstand säulenchromatographisch gereinigt (Laufmittel Dichlormethan). Das Eluat wurde eingeengt und der Rückstand einer Wasserdampfdestillation unterzogen (Siedebereich des Azeotrops mit Wasser: 60-65°C bei p=0,1 mm Hg). Nach der Extraktion des Destillates mit Diethylether, Trocknen der organischen Phase und Einengen der Lösung erhielt man 10.6 g (88 mmol, 36 %) der Titelverbindung. ¹H-NMR (270 MHz, d₆-DMSO): δ = 7.7 (d, 1H), 7.8 (d, 1H), 9.75 (s, 1H).
b) 5-Hydroxymethyl-2-cyanofuran:
   Zu einer Lösung von 30g (0,25 mol) 5-Cyanofuran-2-carbaldehyd in 500 ml absolutem Ethanol wurden bei -30°C portionsweise 2,34 g (62 mmol) Natriumborhydrid gegeben. Man rührte zwei Stunden bei -30°C und brachte die gekühlte Reaktionslösung mit Hilfe einer 5 %igen Zitronensäurelösung in Wasser auf pH 7. Man engte das Reaktionsgemisch im Wasserstrahlvakuum ein, versetzte den Rückstand mit gesättigter Natriumchloridlösung, extrahierte mehrfach mit je 150 ml Diethylether, trocknete die vereinigten organischen Phasen über Magnesiumsulfat, filtrierte das Trockenmittel ab und destillierte das Lösungsmittel im Wasserstrahlvakuum bei Raumtemperatur ab. Man erhielt auf diese Weise 27 g (22 mmol, 88 %) der Titelverbindung als dunkelrotes Öl, das ohne weitere Reinigung in die folgenden Umsetzungen eingesetzt wurde. ¹H-NMR (250 MHz, d₆-DMSO) : δ = 4.4 (m, 2H), 5.6 (bs, 1H), 6.6 (d, 1H), 7.5 (d, 1H).
c) 5-Brommethyl-2-cyanofuran:
   Zu einer Lösung von 15g (121 mol) 5-Hydroxymethyl-2-cyanofuran in 250 ml Tetrahydrofuran wurden 38 g (145 mmol) Triphenylphosphin gegeben. Man kühlte auf -10°C und gab eine Lösung von 48g (145 mmol) Tetrabrommethan in 100 ml Tetrahydrofuran hinzu. Man ließ auf Raumtemperatur erwärmen und rührte drei Stunden lang bei dieser Temperatur. Die Reaktionsmischung wurde am Rotationsverdampfer im Wasserstrahlvakuum eingeengt und der Rückstand säulenchromatographisch gereinigt (Laufmittel Petrolether: Dichlormethan 1:1, R_{f} =0.5). Man erhielt 11,5g der Titelverbindung. ¹H-NMR (250 MHz, d₆-DMSO) : δ = 4.8 (m, 2H), 6.7 (d, 1H), 7.7 (d, 1H).
d) 5-N,N-Bis(tert.-butoxycarbonyl)aminomethyl-2-cyanofuran:
   Eine auf 0°C gekühlte Lösung von 22,9 g (123 mmol) 5-Brommethyl-2-cyanofuran in 400 ml Tetrahydrofuran wurde portionsweise mit 4,0 g (135 mmol) Natriumhydrid (80 %ige Suspension in Mineralöl) versetzt. Anschließend wurde eine Lösung von 29,4 g (135 mmol) Di-tert.-butyl-iminodicarboxylat in 200 ml Tetrahydrofuran hinzugetropft, wobei die Temperatur 5°C nicht überstieg. Man ließ auf Raumtemperatur erwärmen und über Nacht rühren. Da der Umsatz unvollständig war (DC-Kontrolle), wurden über einen Zeitraum von 9 Stunden noch insgesamt 1,2 g Natriumhydrid in drei Portionen nachgegeben. Man erwärmte zur Vervollständigung des Umsatzes noch drei Stunden lang auf 35°C, ließ danach auf Raumtemperatur abkühlen und versetzte langsam mit 600 ml einer gesättigten Ammoniumchloridlösung. Das Lösungsmittel wurde im Wasserstrahlvakuum abdestilliert, der Rückstand mehrere Male mit Essigsäureethylester extrahiert, die vereinigten organischen Phasen mit gesättigter Natriumchloridlösung gewaschen, über Magnesiumsulfat getrocknet und am Rotationsverdampfer eingeengt. Man erhielt 37,3 g eines öligen Rückstandes, der noch Di-tert.-butyl-iminodicarboxylat enthielt und als Rohprodukt in die folgende Umsetzung eingesetzt wurde. ¹H-NMR (250 MHz, d₆-DMSO): δ = 1.40, 1.45 (s, 18H), 4.75 (s, 2H), 6.55 (d, 1H), 7.55 (d, 1H).
e) 5-Aminomethyl-2-cyanofuranhydrochlorid:
   37,3 g 5-N,N-Bis(tert.-butoxycarbonyl)aminomethyl-2-cyanofuran (Rohprodukt aus d), maximal 123 mmol) wurden in 600 ml Essigsäureethylester gelöst und auf 0°C gekühlt. Man sättigte mit Chlorwasserstoffgas, wobei nach 30 min ein weißer Niederschlag ausfiel. Man ließ auf Raumtemperatur kommen, ließ über Nacht rühren, engte die entstandene Suspension anschließend am Rotationsverdampfer ein, rührte den Rückstand mit Diethylether aus, filtrierte vom Lösungsmittel ab und trocknete den festen Rückstand bei Raumtemperatur im Vakuum. Man erhielt 15,1g (77% Ausbeute über zwei Stufen) der Titelverbindung als leicht ockerfarbenes Pulver. ¹H-NMR (250 MHz, d₆-DMSO): δ = 4.15 (bs, 2H), 6.85 (d, 1H), 7.65 (d, 1H), 8.8-9.0 (bs, 3H).

### 5-Aminomethyl-3-cyanofuran

a) 4-Oxopentansäureethylester
   100 g (0,86 Mol) 4-Oxopentansäure, 150 g Ethanol und 1 ml Schwefelsäure wurden in 200 ml Benzol unter Rückfluß erhitzt, bis die Wassertrennung in der Dean-Stark-Falle beendet war. Das gekühlte Reaktionsgemisch wurde mit Wasser, Natriumcarbonatlösung und erneut mit Wasser gewaschen und dann unter Rückfluß mit der Dean-Stark-Falle getrocknet. Als die Abtrennung der Wasserphase beendet war, wurde das Lösungsmittel abdestilliert und der Rückstand unter verringertem Druck destilliert. Sdp. 85-87°C/16 mmHg, Ausbeute 105,5 g (85 %).
b) 4,4-Diethoxypentansäureethylester
   Ein Gemisch aus 171,3 g(1,19 Mol) 4-Oxopentansäureethylester, 207 ml (184,2 g, 1,24 Mol) Triethylorthoformiat, 26 ml absochmlutem Ethanol und 1 g p-Toluolsulfonsäure wurde unter gründlichem Rühren 8 h unter Rückfluß erhitzt und dann im Vakuum destilliert. Es wurden 187,9 g (72,5 %) 4,4-Diethoxypentansäureethylester erhalten, Sdp. 104-106°C/14 mm Hg.
c) 2-Formyllävulinsäureethylester
   Ein Gemisch aus 106,3 g (0,489 Mol) 4,4-Diethoxypentansäureethylester und 80 ml (73,6 g, 0,99 Mol) Ethylformiat wurde unter gründlichem Rühren tropfenweise zu einer Suspension aus 12,7 g (0,55 grammatom) (dünnblättrigem) Natrium in 300 ml wasserfreiem Benzol 3 h bei 10-15°C gegeben. Das Rühren wurde weitere 3 h fortgesetzt, und das Reaktionsgemisch über Nacht stehengelassen. Es wurden 250 ml Wasser unter gründlichem Rühren zugegeben, das noch weitere 15 min fortgeführt wurde. Die Wasserschicht wurde abgetrennt, und die Benzolschicht wurde mit 70 ml Wasser extrahiert. Die vereinigten Wasserextrakte wurden auf einen pH-Wert von 2 angesäuert, mit Ethylacetat (5x50 ml) extrahiert, und die organischen Extrakte wurden über Calciumchlorid getrocknet.
   Die Ethylacetatlösung wurde im Vakuum destilliert, und die Fraktion mit einem Sdp. von 102-110°C/1 mm Hg gesammelt. Diese Fraktion ist ein Gemisch aus 2-Formyllävulinsäureethylester und seinem Diethylketal. Ihr Mischungsverhältnis hängt von der Intensität des Rührvorgangs und der Dauer der Phasentrennung bei der Isolation der Formylierungsprodukte ab.
   Die Benzolschicht wurde ebenfalls über Calciumchlorid getrocknet, das Lösungsmittel wurde entfernt, und der Rückstand wurde unter verringertem Druck destilliert, wonach das entstandene Lävulinsäureethylester-Ketal-Gemisch wie in b) beschrieben anstelle des reinen Lävulinsäureethylesters behandelt wurde. Die Schritte (2) und (3a) wurden so lange wiederholt, bis die nötige Menge 2-Formyllävulinsäureethylester erhalten wurde.
d) 5-Methylfuran-3-carbonsäureethylester
   Das vorstehend genannte Gemisch aus 2-Formyllävulinsäureethylester und seinem Diethylketal wurde in Benzol gelöst, der Katalysator wurde zugesetzt, und die erhaltene Lösung wurde mit einer Dean-Stark-Falle 3-3,5 h unter Rückfluß belassen, bis das Wasser vollständig entfernt war. Danach wurde das Reaktionsgemisch unter verringertem Druck destilliert, wobei 15 g (97 mMol) 5-Methylfuran-3-carbonsäureethylester, Sdp. 97°C/15 mm Hg, erhalten wurden.
e) 5-Methylfuran-3-carbonsäure
   Ein Gemisch aus 31,7 g(206 mMol) 5-Methylfuran-3-carbonsäureethylester, 40 ml 45 %iges Kaliumhydroxid und 100 ml Wasser wurde 4 h unter Rückfluß und unter Rühren erhitzt, dann auf 10°C gekühlt und mit 15 %iger Salzsäure auf einen pH-Wert von 1 angesäuert. Das entstandene Reaktionsgemisch wurde 2 h bei dieser Temperatur belassen, das Präzipitat wurde abfiltriert und bei 45-50°C bis zur Gewichtskonstanz getrocknet, wobei 23,7 g (188 mMol, 91%) 5-Methylfuran-3-carbonsäure erhalten wurden.
f) 5-Methylfuran-3-carbonsäurechlorid
   Zu einer Suspension aus 23,7 g (188 mMol) 5-Methylfuran-3-carbonsäure in 100 ml Benzol wurden 39,2 g (188 mMol) Phosphor(V)-chlorid in kleinen Mengen unter Rühren gegeben. Man beobachtete eine beträchtliche Wärmefreisetzung und Chlorwasserstoff entwicklung. Das entstandene Gemisch wurde 4 h unter Rückfluß und Rühren belassen und dann im Vakuum destilliert. Es wurden 24,7 g (171 mMol, 91 %) Säurechlorid erhalten, Sdp. 79°C/12 mm Hg.
g) 5-Methylfuran-3-carbonsäureamid
   Es wurden 24,7 g (171 mMol) 5-Methylfuran-3-carbonsäurechlorid tropfenweise unter Rühren zu einem Gemisch aus 80 ml einer 25%igen Ammoniumhydroxidlösung und 80 ml Benzol bei 25-40°C gegeben. Das entstandene Gemisch wurde 3 h gerührt und über Nacht stehen gelassen. Am darauffolgenden Tag wurden weiße Amidkristalle abfiltriert, mit kaltem Wasser gewaschen und bei 40-45°C bis zur Gewichtskonstanz getrocknet. Ausbeute 19,7 g (158 mMol, 92 %), Schmp. 158°C.
h) 5-Methyl-3-cyanofuran
   Zu einer Suspension aus 19,7 g (158 mMol) 5-Methylfuran-3-carbonsäureamid in 100 ml Benzol wurden 32,9 g Phosphor(V)-chlorid in kleinen Mengen bei 30-40°C gegeben. Das entstandene Gemisch wurde bis zur Klärung (3,5-4 h) unter Rückfluß und unter Rühren belassen und anschließend unter verringertem Druck destilliert. Die Fraktion mit einem Sdp. von 79-140°C/15 mm Hg wurde gesammelt. Die zweite Destillation ergab 12,7 g (119 mMol, 75 %) der Titelverbindung, Sdp. 79-80°C/15 mm Hg.
i) 5-Brommethyl-3-furancarbonitril
   Es wurden 12,7 g (119 mMol) 5-Methyl-3-cyanofuran in 100 ml Tetrachlorkohlenstoff gelöst, und 22 g (122 mMol) NBS und 12 g (73 mMol) AIBN wurden zugegeben. Das entstandene Gemisch wurde unter gründlichem Rühren auf 70°C erhitzt, als die exotherme Reaktion begann. Nach Beendigung der Wärmefreisetzung wurde das Reaktionsgemisch 3 h bei 80°C gerührt, dann auf Raumtemperatur gekühlt, das entstandene Succinimid wurde abfiltriert und auf dem Filter mit Tetrachlorkohlenstoff (2x15 ml) gewaschen.
   Das Lösungsmittel wurde bei verringertem Druck entfernt, und der Rückstand wurde im Vakuum destilliert, wobei 12,7 g (86 mMol, 57%) 5-Brommethyl-3-furancarbonitril, Sdp. 105°C/l mm Hg, erhalten wurden. Gemäß 1H-NMR-Spektrum enthält es Verunreinigungen, die bei δ 1,3 und 2,2 ppm Signale erzeugen. Nach der zweiten Destillation war ihr Gehalt zufriedenstellend niedriger, jedoch wurden etwa 15 % des Produktes eingebüßt. 5-Brommethyl-3-furancarbonitril ist eine weiße kristalline Substanz, Schmp. 40-45°C. 1H-NMR (CDCl₃, ppm): 4,41 (2H, CH₂), 6,58 (1H, H4), 7,92 1H, H2); 13C-NMR (CDCl₃, ppm): 20,86 (CH₂), 99,03 (CN oder weniger wahrscheinlich C3), 109,97 (C4), 112, 27 (C3 (oder CN)), 149,84 (C2), 152, 32 (C5).
   Das Reaktionsprodukt ist stark reizend und muß deshalb mit äußerster Vorsicht gehandhabt werden.
   Die Synthese von 5-N,N-Bis(tert.-butoxycarbonyl)aminomethyl-3-cyanofuran erfolgte analog zur Synthese des 5-N,N-Bis(tert.-butoxycarbonyl)aminomethyl-2-cyanofurans. Die anschließende Abspaltung der tert.-Butoxycarbonylgruppen wurde in einer gesättigten Lösung von Chlorwasserstoff in Chloroform durchgeführt.

### 2-Amidino-5-(N-Boc-aminomethyl)-1-methylpyrrol Hydroacetat

a) 5-Cyano-1-methylpyrrol-2-carbaldehyd
   1-Methylpyrrol kann durch Umsetzung mit Chlorsulfonylisocyanat und Dimethylformamid in Acetonitril in 2-Cyano-1-methylpyrrol überführt werden (siehe z.B. C.E. Loader et al. Can. J. Chem. (1981), 59, 2673-6).
   Diisopropylamin (17,5 ml, 124,38 mmol) wurde unter Stickstoff in THF (100 ml) vorgelegt. Bei -78°C wurde n-Butyllithiumlösung in Hexan (15%ig, 75,9 ml, 124,38 mmol) zugetropft. Anschließend wurde 45 min bei -20°C gerührt und dann wieder auf -78°C abgekühlt. Bei dieser Temperatur wurde eine Lösung von N-Methylpyrrol-2-carbonitril (12 g, 113,07 mmol) in THF (50 ml) zugetropft. Nach 45 min Rühren bei -78°C wurde DMF (43,9 ml, 546,46 mmol) zugetropft und weitere 2 h bei dieser Temperatur gerührt. Nach Zusatz von Zitronensäuremonohydrat (20,56 g) wurde auf Raumtemperatur erwärmt und mit Wasser (112 ml) versetzt. Das THF wurde abrotiert, die wässrige Phase wurde mit Natriumchlorid gesättigt und mit Diethylether (3 x 200 ml) extrahiert. Die vereinten organischen Phasen wurden mit gesättigter Natriumchloridlösung gewaschen und über Natriumsulfat getrocknet. Das Lösungsmittel wurde abrotiert und das Rohprodukt mittels Flashchromatographie gereinigt (Kieselgel, Dichlormethan). Ausbeute: 8,25 g (54 %). 1H-NMR (CDCl₃) δ = 4,1 (s, 3H) , 6,8 (d, 1H), 6,9 (d, 1H), 9,7 (s, 1H).
b) 5-Hydroxymethyl-1-methylpyrrol-2-carbonitril
   Das gemäß a) erhaltene Produkt (8,2 g, 61,1 mmol) wurde in Ethanol (200 ml) gelöst und bei -10°C mit Natriumborhydrid (2,31 g, 61,13 mmol) versetzt. Nach 1,5 h Rühren bei 0-5°C wurde das Lösungsmittel abrotiert und der Rückstand mit Eiswasser und 20 %iger Natriumhydrogensulfatlösung versetzt. Die wässrige Phase wurde mit Essigsäureethylester extrahiert. Die vereinigten organischen Phasen wurden mit gesättigter Natriumhydrogencarbonatlösung und Wasser neutral gewaschen und über Natriumsulfat getrocknet. Das Lösungsmittel wurde abrotiert und das Rohprodukt mittels Flashchromatographie gereinigt (Kieselgel, Dichlormethan/Methanol=97.5/2.5). Ausbeute: 7,6 g (91%).
   1H-NMR (CDCl₃) δ = 1,9 (t, 1H), 3,75 (s, 3H), 4,6 (d, 2H), 6,1 (d, 1H), 6, 7 (d, 1H).
c) 5-Azidomethyl-1-methylpyrrol-2-carbonitril
   Das gemäß b) erhaltene Produkt (7,5 g, 55,08 mmol) wurde in DMF (220 ml) gelöst und bei 0°C mit Triphenylphosphin (43,34 g, 165,25 mmol) versetzt. Nach 5 min Rühren bei dieser Temperatur wurde Tetrabrommethan (54,8 g, 165,25 mmol) zugegeben. Anschließend wurde 30 min bei 0°C und 1,5 h bei Raumtemperatur gerührt. Nach Abkühlen auf 0°C wurde mit Natriumazid (4,37 g, 67,21 mmol) versetzt. Anschließend wurde 4,5 h bei Raumtemperatur gerührt. Bei 0°C wurde gesättigte Natriumchloridlösung zugetropft und der Ansatz mit Essigsäureethylester verdünnt. Die organische Phase wurde abgetrennt und die wässrige Phase mit Diethylether extrahiert. Die vereinigten organischen Phasen wurden mit Wasser gewaschen und über Natriumsulfat getrocknet. Das Lösungsmittel wurde abrotiert und das Rohprodukt mittels Flashchromatographie gereinigt (Kieselgel, Essigsäureethylester/Hexan=1/20). Ausbeute: 5,6 g (63 %).
   1H-NMR (CDCl₃) δ = 3,75 (s, 3H), 4,35 (s, 2H), 6,2 (d, 1H), 6,7 (d, 1H).
d) 5-Aminomethyl-1-methylpyrrol-2-carbonitril
   Das gemäß c) erhaltene Produkt (4,71 g, 29,25 mmol) wurde in Methanol (100 ml) gelöst und mit Palladium auf Kohle (10 %ig, 1 g) versetzt. Anschließend wurde 4 h unter 1 Atmosphäre mit Wasserstoff hydriert. Der Katalysator wurde über Celite® abfiltriert und das Filtrat einrotiert. Der Rückstand wurde mit Dichlormethan/Diethylether = 1/1 ausgerührt. Das Produkt wurde abgesaugt und im Vakuumtrockenschrank bei 35°C getrocknet. Ausbeute: 2,7 g (68%).
   1H-NMR (CDCl₃) δ = 3,75 (s, 3H), 3,85 (s, 2H), 6,05 (d, 1H), 6,7 (d, 1H).
e) 5-(N-Boc-Aminomethyl)-1-methylpyrrol-2-carbonitril
   Das gemäß d) erhaltene Produkt (2,7 g, 19,97 mmol) wurde in Dichlormethan (50 ml) gelöst und mit Triethylamin (2,8 ml, 19,97 mmol) versetzt. Anschließend wurde eine Lösung von Ditertiärbutyldicarbonat (4,36 g, 19,97 mmol) in Dichlormethan (30 ml) zugetropft. Nach 2 h Rühren bei Raumtemperatur wurde mit Wasser versetzt und die wässrige Phase mit Dichlormethan extrahiert. Die vereinigten organischen Phasen wurden über Natriumsulfat getrocknet und einrotiert. Das Rohprodukt wurde ohne weitere Reinigung in die folgende Reaktion eingesetzt. Ausbeute: 4,4 g (94 %).
   1H-NMR (CDCl₃) δ = 1,45 und 1,55 (jeweils s, zusammen 9H), 3,7 (s, 3H), 4,3 (d, 2H), 4,7 (sbr, 1H), 6,05 (d, 1H), 6,7 (d, 1H).
f) 5-(N-Boc-Aminomethyl)-1-methylpyrrol-2-hydroxyamidin
   Das gemäß e) erhaltene Produkt (4,3 g, 18,27 mmol) wurde in Methanol/Dichlormethan (100 ml, 1/1) gelöst und mit Hydroxylaminhydrochlorid (3,17 g, 45,61 mmol) versetzt. Anschließend wurde Ethyldiisopropylamin (19,1 ml, 109,65 mmol) bei Raumtemperatur zugetropft. Nach 12 h Rühren bei 40°C wurde das Lösungsmittel abrotiert, der Rückstand mit Wasser versetzt, mit Essigsäure auf pH 5 angesäuert und mit Dichlormethan und Essigsäureethylester extrahiert. Die vereinigten organischen Phasen wurden über Natriumsulfat getrocknet und einrotiert. Das Rohprodukt wurde mittels Flashchromatographie gereinigt (Kieselgel, Dichlormethan/Methanol = 95/5). Ausbeute: 3,4 g (69%).
   1H-NMR (CDCl₃) δ = 1,4 (s, 9H), 3,7 (s, 3H), 4,3 (d, 2H), 4,7-4,9 (m, 3H), 6,05 (d, 1H), 6,3 (d, 1H), 7,3 (sbr, 1H).
g) 2-Amidino-5-(N-Boc-aminomethyl)-1-methylpyrrol Hydroacetat
   Das gemäß f) erhaltene Produkt (3,4 g, 12,67 mmol) wurde in Methanol (150 ml) gelöst und mit Essigsäure (1,45 ml, 25,31 mmol) und Raney-Nickel (421 mg) versetzt. Anschließend wurde 5 h bei 50°C unter 1 Atmosphäre Wasserstoff hydriert. Nach Abkühlen auf Raumtemperatur wurde der Katalysator über Celite® abfiltriert und das Filtrat eingeengt. Das so erhaltene Produkt wurde ohne weitere Reinigung in die folgende Reaktion eingesetzt. Ausbeute: 3,7 g (94%).
   FAB-MS (M+H⁺): 253.

### 2-Amidino-4-(N-Boc-aminomethyl)-1-methylpyrrol Hydroacetat

a) 5-Cyano-1-methylpyrrol-3-carbaldehyd
   Aluminiumtrichlorid (24,24 g, 180,86 mmol) wurde in Nitromethan/Dichlormethan (1/1, 320 ml) gelöst, auf -20°C gekühlt und mit 1-Methylpyrrol-2-carbonitril (8 g, 75,36 mmol) versetzt. Anschließend wurde α,α-Dichlordimethylether (10,4 g, 90,43 mmol) gelöst in Dichlormethan (42 ml) zugetropft. Nach 4 h Rühren bei 0°C wurde der Ansatz auf Eis gegossen (200 g). Die wässrige Phase wurde mit Diethylether extrahiert. Die vereinigten organischen Phasen wurden mit gesättigter Natriumhydrogencarbonatlösung, Wasser und gesättigter Natriumchloridlösung neutral gewaschen. Nach Trocknen über Natriumsulfat wurde das Lösungsmittel abrotiert. Das Rohprodukt wurde ohne weitere Reinigung in die folgenden Reaktionen eingesetzt. Ausbeute: 9,2 g (91 %).
   1H-NMR (CDCl₃) δ = 3,8 (s,3H); 7,2 (s, 1H); 7,4 (s, 1H); 9,85 (s, 1H).
b) 4-Aminomethyl-1-methylpyrrol-2-carbonitril wurde ausgehend von 5-Cyano-1-methylpyrrol-3-carbaldehyd analog der Synthese für 5-Aminomethyl-1-methylpyrrol-2-carbonitril dargestellt. Die Reduktion des 4-Azidomethyl-1-methylpyrrol-2-carbonitrils erfolgte jedoch vorteilhafter Weise über eine Staudinger Reaktion (s. z.B. S. Nagarajan et al. J. Org. Chem. 1987, 52, 5044-6).
c) 2-Amidino-4-(N-Boc-aminomethyl)-1-methylpyrrol Hydroacetat wurde ausgehend von 4-Aminomethyl-1-methylpyrrol-2-carbonitril analog der Synthese von 2-Amidino-5-(N-Boc-aminomethyl)-1-methylpyrrol Hydroacetat dargestellt.
   FAB-MS (M+H⁺) : 253.

### 4-Amidino-2-(N-Boc-aminomethyl)-1-methylpyrrol Hydroacetat

a) 4-Cyano-1-methylpyrrol-2-carbaldehyd
   1-Methylpyrrol-2-carbaldehyd (10 g, 91,6 mmol) wurde in Acetonitril (100 ml) gelöst und auf -45°C gekühlt. Chlorsulfonylisocyanat (38,9 g, 274,9 mmol) in Acetonitril (40 ml) wurde über 40 min zugetropft. Anschließend wurde 12 h bei Raumtemperatur gerührt. Nach tropfenweiser Zugabe von Dimethylformamid (35 ml) wurde 1 h auf 50°C erwärmt. Nach Abkühlen auf Raumtemperatur wurde das Reaktionsgemisch auf Eis (200 ml) und 2N Natronlauge (286 ml) gegeben. Der gebildete Niederschlag wurde abgesaugt. Das Filtrat wurden mit Diethylether extrahiert. Die vereinigten Etherphasen wurden mit verdünnter Natriumhydrogencarbonatlösung und Wasser neutral gewaschen und über Natriumsulfat getrocknet. Das Lösungsmittel wurde im Wasserstrahlvakuum abdestilliert und der Rückstand mit dem zuvor gewonnenen Niederschlag vereint. Umkristallisation aus Petrolether ergab 4-Cyano-1-methylpyrrol-2-carbaldehyd (4,3 g) (siehe z.B. C.E. Loader et al. Can. J. Chem. (1981), 59, 2673-6)
   1-H-NMR (CDCl₃) δ = 4,0 (s,3H); 7,2 (s, 1H); 7,3 (s, 1H); 9,6 (s, 1H).
   ¹³-C-NMR (CDCl₃) δ = 37,4; 94,1; 114,7; 125,8; 132,2; 135,8; 179,7.
b) 5-Aminomethyl-1-methylpyrrol-3-carbonitril wurde ausgehend von 4-Cyano-1-methylpyrrol-2-carbaldehyd analog der Synthese für 5-Aminomethyl-1-methylpyrrol-2-carbonitril dargestellt.
   1H-NMR (DMSO-d₆) δ = 3,6 (s, 3H), 3,8 (s, 2H), 4,2 (sbr, 2H), 6,4 (s, 1H), 7,6 (s, 1H).
c) 3-Amidino-5-(N-Boc-aminomethyl)-1-methylpyrrol Hydroacetat wurde ausgehend von 5-Aminomethyl-1-methylpyrrol-3-carbonitril analog der Synthese von 2-Amidino-5-(N-Boc-aminomethyl)-1-methylpyrrol Hydroacetat dargestellt.
   FAB-MS (M+H⁺) : 253.

### 5-Aminomethyl-3-cyano-1,2,4-oxadiazol Hydrochlorid

a) N-Boc-5-Aminomethyl-3-cyano-1,2,4-oxadiazol
   N-Boc-5-Aminomethyl-1,2,4-oxadiazol-2-carbonsäureethylester (S. Borg et al. J. Org. Chem. 1995, 60, 3112-20) wurde in Methanol (50 ml) gelöst. In diese Lösung wurde bei -10°C bis RT Ammoniak bis zur vollständigen Umsetzung eingeleitet. Das Lösungsmittel wurde abrotiert. Das so erhaltene Rohprodukt wurde in Dichlormethan (70 ml) gelöst und bei -5°C mit Diisopropylethylamin (2,9 ml, 16,55 mmol) versetzt. Anschließend wurde Trifluoressigsäureanhydrid (1,06 ml, 7,61 mmol) gelöst in Dichlormethan (10 ml) zugetropft. Nach 1,5 h Rühren bei 0°C wurde der Ansatz mit Dichlormethan verdünnt, 2x mit gesättigter Natriumhydrogencarbonatlösung, 2x mit 5%iger Zitronensäurelösung und 1x mit gesättigter Natriumchloridlösung gewaschen und dann über Natriumsulfat getrocknet. Das Lösungsmittel wurde abrotiert und das Rohprodukt chromatographisch gereinigt (Kieselgel, Dichlormethan:Methanol = 97,5:2,5). Ausbeute: 1,2 g (80 %).
b) 5-Aminomethyl-3-cyano-1,2,4-oxadiazol Hydrochlorid
   Das gemäß a) erhaltene Produkt (0,9 g, 4,0 mmol) wurde in Dichlormethan (45 ml) gelöst und bei RT mit 4 M Salzsäure in Dioxan (3,9 ml, 15,61 mmol) versetzt. Nach 16 h Rühren bei RT wurde das Lösungsmittel abrotiert. Ausbeute: 645 mg (100 %).
   1-H-NMR (DMSO-d₆) δ = 4.6 (s, 2H), 9.2 (s, 3H).

### N-Methyl-5-aminomethyl-pyrazol-3-carbonsäureamid

a) N-Methyl-5-amido-pyrazol-3-carbonsäuremethylester
   N-Methyl-3-methoxycarbonyl-pyrazol-5-carbonsäurechlorid (dargestellt aus 3,7 g, 20,09 mmol N-Methyl-3-methoxycarbonyl-3-carbonsäure, J. Org. Chem. 1989, 54, 428) wurde in Toluol gelöst und auf -10°C gekühlt. Anschließend wurde bei -10°C bis 0°C Ammoniak bis zur vollständigen Umsetzung eingeleitet. Das Lösungsmittel wurde abrotiert. Der Rückstand wurde in Ethanol aufgenommen. Nach 15 min Rühren wurde das Ethanol abrotiert, der Rückstand in warmen Wasser gelöst und durch Abkühlen auf 0°C gefällt. Der Niederschlag wurde abgesaugt, mit Aceton gewaschen und im Vakuum bei 45°C getrocknet. Ausbeute: 1,5 g (41 %).
b) N-Methyl-5-cyano-pyrazol-3-carbonsäuremethylester
   Das gemäß a) erhaltene Produkt (1,5 g, 8,19 mmol) wurden in Dichlormethan (20 ml) aufgenommen. Bei -10°C wurde mit Diisopropylethylamin (3,85 ml, 22,11 mmol) versetzt und bei dieser Temperatur eine Lösung von Trifluoressigsäureanhydrid (1,3 ml, 9,44 mmol) in Dichlormethan (5 ml) über 45 min zugetropft. Anschließend wurde noch 1 h bei 0°C gerührt. Der Ansatz wurde mit Dichlormethan verdünnt, 2x mit gesättigter Natriumhydrogencarbonatlösung, 2x mit 5 %iger Zitronensäurelösung und 1x mit gesättigter Natriumchloridlösung gewaschen. Nach Trocknen über Natriumsulfat wurde das Lösungsmittel abrotiert. Ausbeute: 1,35 g (100 %).
   EI-MS (M+): 165.
c) N-Methyl-5-cyano-pyrazol-3-carbonsäureamid
   Das gemäß b) erhaltene Produkt (1,35 g, 8,19 mmol) wurde in Methanol (50 ml) vorgelegt und auf -10°C gekühlt. Anschließend wurde über 8 h Ammoniak eingeleitet. Nach 12 h Rühren bei RT hatte das Edukt abreagiert. Das ausgefallene Produkt wurde abgesaugt, mit kaltem Methanol gewaschen und im Vakuum getrocknet. Ausbeute: 1,22 g (100 %).
   1-H-NMR (DMSO-d₆) δ = 4. 0 (s, 3H), 7.4 (s, 1H), 7.5 (s, 1H), 7.8 (s, 1H).
d) N-Methyl-5-aminomethyl-pyrazol-3-carbonsäureamid
   Das gemäß c) erhaltene Produkt (0,4 g, 2,66 mmol) wurde in Essigsäure (30 ml) gelöst und mit 10 % Palladium auf Kohle (78 mg) versetzt. Anschließend wurde bei RT unter Normaldruck bis zum vollständigen Umsatz hydriert. Der Katalysator wurde über Celite® abfiltriert und das Lösungsmittel abrotiert. Ausbeute: 0,4 g (100 %).
   EI-MS (M+): 154.

### Beispiel 1: N-(Hydroxycarbonyl-methylen)-(D)-cyclohexylalanyl-3,4-dehydroprolyl-5-(2-amidino)-thienylmethylamid-hydroacetat

a) 3,4-Dehydroprolyl-5-(2-cyano)-thienylmethylamid:
   Boc-3,4-Dehydroprolin (5 g, 23,4 mmol) und 5-Aminomethyl-2-cyanothiophen-hydrochlorid (4,5 g, 25,8 mmol) wurden in Dichlormethan (25 ml) gelöst und bei 0°C mit Ethyldiisopropylamin (28 ml, 163,8 mmol) mit einer 50 %igen Lösung von Propanphosphonsäureanhydrid in Essigsäureethylester (24,8 ml, 117 mmol) versetzt. Nach 1 h Rühren bei 0°C wurde auf Raumtemperatur erwärmt und 12 h bei Raumtemperatur nachgerührt. Die Reaktionsmischung wurde mit Dichlormethan verdünnt, mit Natriumhydrogensulfatlösung (4x), Natriumhydrogencarbonatlösung (3x) und gesättigter Natriumchloridlösung (1x) gewaschen. Nach Trocknen über Natriumsulfat und Abfiltrieren des Trockenmittels wurde das Lösungsmittel im Wasserstrahlvakuum abdestilliert. Der Rückstand wurde zur Abspaltung der Boc-Gruppe in Dichlormethan (95 ml) versetzt, bei Raumtemperatur gerührt, zur Trockne eingedampft, zweimal mit Dichlormethan kodestilliert, erneut eingeengt und säulenchromatographisch gereinigt. Man erhielt 6,6 g des gewünschten Produktes welches noch leicht lösungsmittelhaltig war.
b) N-(tert.Butoxycarbonyl-methylen)-(N-Boc)-(D)-cyclohexylalanyl-3,4-dehydroprolyl-5-(2-cyano)-thienylmethylamid:
   t-BuO₂C-CH₂-BOC-(D)-Cha-OH (7,3 g, 18,98 mmol) und H-Pyr-NH-CH₂-5-(2-CN)-thioph-hydrochlorid (5,12 g, 18,98 mmol) wurden in Dichlormethan (100 ml) gelöst und mit Ethyldiisopropylamin (12,26 g, 94,9 mmol) versetzt. Das Reaktionsgemisch wurde auf 0°C gekühlt und tropfenweise mit einer 50 %igen Lösung von Propanphosphonsäureanhydrid in Essigsäureethylester (20 ml) versetzt. Nach 3 h Rühren bei 0 - 10°C wurde mit Dichlormethan (100 ml) verdünnt und mit verd. Natriumhydrogensulfatlösung (3x), gesättigter Natriumhydrogencarbonatlösung (2x) und Wasser (1x) gewaschen. Nach Trocknen über Natriumsulfat wurde das Lösungsmittel nach Abtrennung des Trockenmittels im Wasserstrahlvakuum abdestilliert. Man erhielt 12,47 g eines leicht bräunlichen Öls.
c) N-(tert.Butoxycarbonyl-methylen)-(N-Boc)-(D)-cyclohexylalanyl-3,4-dehydroprolyl-5-(2-amidothiocarbonyl)-thienylmethylamid:
   Das gemäß b) erhaltene Produkt wurde in Pyridin (70 ml) und Triethylamin (12 ml) gelöst. Die Reaktionsmischung wurde auf 0°C gekühlt und mit Schwefelwasserstoff gesättigt (die Lösung färbte sich grün). Anschließend wurde 48 h bei Raumtemperatur nachgerührt. Der überschüssige Schwefelwasserstoff wurde mit Stickstoff verdrängt und das Lösungsmittel im Wasserstrahlvakuum abdestilliert. Der Rückstand wurde in Diethylether (200 ml) gelöst, mit verdünnter Natriumhydrogensulfatlösung (2x), gesättigter Natriumhydrogencarbonatlösung (2x) und Wasser (1x) gewaschen. Nach Trocknen über Natriumsulfat wurde das Lösungsmittel im Wasserstrahlvakuum abdestilliert. Das Rohprodukt (12,6 g) wurde mittels Flashchromatographie (Kieselgel, Gradient von Dichlormethan bis Dichlormethan : Methanol = 40 : 1) gereinigt. Man erhielt 12,1 g des gewünschten Produktes, welches noch leicht lösungsmittelhaltig war.
d) N-(tert.Butoxycarbonyl-methylen)-N-Boc)-(D)-cyclohexylalanyl-3,4-dehydroprolyl-5-(2-S-methylimidocarbonyl)-thienylmethylamid-hydroiodid:
   Das gemäß c) erhaltene Produkt wurde in Dichlormethan (120 ml) gelöst und mit Methyliodid (16,24 g, 114,38 mmol) versetzt. Nach 12 h Rühren bei Raumtemperatur wurde das Lösungsmittel im Wasserstrahlvakuum abdestilliert. Man erhielt 14,6 g eines gelblichen Öls.
e) N-(tert.Butoxycarbonyl-methylen)-(N-Boc)-(D)-cyclohexylalanyl-3,4-dehydroprolyl-5-(2-amidino)-thienylmethylamidhydroacetat.
   Das gemäß d) erhaltene Rohprodukt wurde in Acetonitril (90 ml) gelöst und mit Ammoniumacetat (2,94 g, 38,12 mmol) versetzt. Nach 2 h Rühren bei Raumtemperatur und 1,5 h bei 40°C wurde mit 10 %iger Ammoniumacetatlösung in Methanol (14,65 g, 19,05 mmol) versetzt. Man rührte noch 4,5 h bei 50°C und destillierte das Lösungsmittel im Wasserstrahlvakuum ab. Der Rückstand wurde mit Dichlormethan versetzt, die Salze abgesaugt und das Filtrat eingeengt. Der Rückstand wurde über einen Ionentauscher (Fluka, Bestell-Nr. 00402) in das AcetatSalz überführt, wodurch man 11,15 g eines gelblichen Öls erhielt.
f) N-(Hydroxycarbonyl-methylen)-(D)-cyclohexylalanyl-3,4-dehydroprolyl-5-(2-amidino)-thienylmethylamid-hydroacetat
   Das gemäß e) erhaltene Produkt wurde in Dichlormethan (175 ml) gelöst und tropfenweise mit etherischer Salzsäurelösung (38,3 ml) versetzt. Nach 2 h Rühren bei Raumtemperatur wurde das Lösungsmittel im Wasserstrahlvakuum abdestilliert. Der Rückstand wurde mit Dichlormethan versetzt und das Lösungsmittel im Wasserstrahlvakuum abdestilliert (2x). Das Rohprodukt (9,35 g) wurde über einen Ionenaustauscher (Fluka, Bestell-Nr. 00402) und anschließende Flashchromatographie (Kieselgel, Gradient von Dichlormethan : Methanol : 4 : 1 über Dichlormethan : Methanol : 50 %ige Essigsäure : 40 : 10 : 2 bis Dichlormethan : Methanol : 50 %ige Essigsäure = 35 : 15 : 5) gereinigt. Das so erhaltene Produkt wurde in Wasser gelöst. Man filtrierte von unlöslichen Bestandteilen ab und lyophilisierte das Filtrat. Dabei fielen 5,55 g als amorpher weißer Feststoff an.
   FAB-MS (m+H⁺) : 462

### Analog Beispiel 1 wurden hergestellt:

### Beispiel 2:

N-(Hydroxycarbonyl-ethylen)-(D)-cyclohexylalanyl-3,4-dehydroprolyl-5-(2-amidino)-thienylmethylamid-hydroacetat

FAB-MS (M+H⁺) : 476

Die Darstellung erfolgte ausgehend von N-(tert.-Butoxycarbonylethylen)-(N-Boc)-(D)-cyclohexylalanin und 3,4-Dehydroprolyl-5-(2-cyano)-thienylmethylamid über mehrere Stufen analog Beispiel 1.

### Beispiel 3:

N-(Hydroxycarbonyl-methylen)-(D)-cyclohexylglycyl-3,4-dehydroprolyl-5-(2-amidino)-thienylmethylamid-hydroacetat:

FAB-MS (M+H⁺): 448

Die Darstellung erfolgte ausgehend von N-(tert.-Butoxycarbonylmethylen)-(N-Boc)-(D-cyclohexylglycin und 3,4-Dehydroprolyl-5-(2-cyano)-thienylmethylamid über mehrere Stufen analog Beispiel 1.

### Beispiel 4:

N- (Hydroxycarbonyl-methylen) - (D)-cyclohexylalanyl-3,4-dehydroprolyl-5-(2-amidino-3,4-dimethyl)-thienylmethylamid-hydroacetat:

FAB-MS (M+H⁺) : 490

Die Darstellung erfolgte ausgehend von 5-Aminomethyl-3,4-dimethyl-thiophen-2-carbonsäureamid durch Kupplung mit Boc-3,4-dehydroprolin zu Boc-3,4-dehydroprolyl-5-(2-carbamoyl-3,4-dimethyl)-thienylmethylamid analog Beispiel 1. Nach Abspaltung der Boc-Schutzgruppe wurde dieser Baustein mit N-(tert.-Butoxycarbonyl-methylen)-(N-Boc)-(D)-cyclohexylalanin analog Beispiel 1 gekuppelt. Die Dehydratisierung der Amid zur Nitrilfunktion wurde folgendermaßen durchgeführt:

4,8 g (7,42 mmol) N-(tert. Butoxycarbonyl-methylen)-(N-Boc)-(D) -cyclohexylalanyl-3,4-dehydroprolyl-5-(2-carbamoyl-3,4-dimethyl)-thienylmethyl-amid wurden in 60 ml Methylenchlorid gelöst, mit 3,83 g (29,64 mmol) Diisopropylethylamin versetzt und auf 0°C abgekühlt. Dazu tropfte man 2;8 g Trifluoressigsäureanhydrid in 3 ml Methylenchlorid langsam zu und rührte zwei Stunden bei 0-5°C nach. Danach wurde mit 60 ml Methylenchlorid verdünnt und die Reaktionsmischung nacheinander dreimal mit 20 ml 20 %iger Zitronensäure, zweimal mit 20 ml gesättigter Natriumhydrogencarbonatlösung, zweimal mit gesättigter Kochsalzlösung gewaschen, die Methylenchloridphase über Natriumsulfat getrocknet und im Vakuum einrotiert. Man erhielt 5,35 g noch lösungsmittelhaltiges N-(tert.-Butoxycarbonyl-methylen)-(N-Boc)-(D)-cyclohexylalanyl-3,4-dehydroprolyl-5-(2-cyano-3,4-dimethyl)-thienylmethylamid, welches direkt in die nachfolgende Stufe eingesetzt wurde.

Die Umsetzung der Nitrilfunktion in die Amidingruppe und die anschließende Schutzgruppenabspaltung erfolgte in Analogie zu Beispiel 1.

### Beispiel 5:

N-(Hydroxycarbonyl-methylen)-(D)-cyclohexylalanyl-3,4-dehydroprolyl-5-(3-amidino)-thienylmethylamid-hydroacetat:

FAB-MS (M+H⁺) : 462

Die Darstellung erfolgte analog Beispiel 1, wobei anstelle von 5-Aminomethyl-2-cyanothiophen-hydrochlorid 5-Aminomethyl-3-cyanothiophen-hydrochlorid eingesetzt wurde.

### Beispiel 6:

N-(Hydroxycarbonyl-methylen)-(D)-cyclohexylalanyl-3,4-dehydroprolyl-4-(2-amidino)-thienylmethylamid-hydroacetat:

FAB-MS (M+H⁺): 462

Die Darstellung erfolgte analog Beispiel 1, wobei anstelle von 5-Aminomethyl-2-cyanothiophen-hydrochlorid 4-Aminomethyl-2-cyanothiophen-hydrochlorid eingesetzt wurde.

### Beispiel 7a:

N-(Hydroxycarbonyl-methylen)-(D)-cyclohexylalanyl-(D)-4,5-dehydropipecolyl-5-(2-amidino)-thienylmethylamidhydroacetat:

FAB-MS (M+H⁺): 476

### Beispiel 7b:

N-(Hydroxycarbonyl-methylen)-(D)-cyclohexylalanyl-4,5-dehydropipecolyl-5-(2-amidino)-thienylmethylamidhydroacetat

FAB-MS (M+H⁺): 476

Die Darstellung erfolgte analog Beispiel 1, wobei anstelle von Boc-3,4-Dehydroprolin racemische Boc-(D,L)-4,5-dehydropipecolinsäure eingesetzt wurde. Auf der Stufe N-(tert. Butoxycarbonylmethylen)-(N-Boc)-(D)-cyclohexylalanyl-(D,L)-4,5-dehydropipecolyl-5-(2-cyano)-thienylmethylamid konnten die beiden diastereomeren Verbindungen mittels Chromatographie (Kieselgel, Cyclohexan/Essigester 7:3) getrennt werden. Beide Diastereomere wurden anschließend analog Beispiel 1 in die Endprodukte überführt.

### Beispiel 8:

N-(Hydroxycarbonyl-methylen)-(D)-cyclohexylglycyl-3,4-dehydroprolyl-5-(3-amidino)-thienylmethylamid-hydroacetat:

FAB-MS (M+H⁺): 448

Die Darstellung erfolgte analog Beispiel 1, wobei von den Edukten 5-Aminomethyl-3-cyanothiophen und N-(tert.butoxycarbonylmethylen)-N-Boc-(D)-cyclohexylglycyl-3,4-dehydroprolin ausgegangen wurde.

### Beispiel 9:

N-(Hydroxycarbonyl-methylen)-(D)-cyclohexylglycyl-3,4-dehydroprolyl-4-(2-amidino)-thienylmethylamid-hydroacetat:

FAB-MS (M+H⁺): 448

Die Darstellung erfolgte analog Beispiel 1, wobei von den Edukten 4-Aminomethyl-2-cyanothiophen und N-(tert.butoxycarbonylmethylen)-N-Boc-(D)-cyclohexylglycyl-3,4-dehydroprolin ausgegangen wurde.

### Beispiel 10:

N-(Hydroxycarbonyl-methylen)-(D)-cyclohexylalanyl-3,4-dehydroprolyl-5-(2-amidino)-furanylmethylamid-hydroacetat:

FAB-MS (M+H⁺) : 446

Die Darstellung erfolgte analog Beispiel 1, wobei anstelle von 5-Aminomethyl-2-cyanothiophen-hydrochlorid 5-Aminomethyl-2-cyanofuran-hydrochlorid eingesetzt wurde.

### Beispiel 11:

N-(Hydroxycarbonyl-ethylen)-(D)-cyclohexylalanyl-3,4-dehydroprolyl-5-(2-amidino)-furanylmethylamid-hydroacetat:

FAB-MS (M+H⁺): 460

Die Darstellung erfolgte analog Beispiel 1, wobei anstelle von 5-Aminomethyl-2-cyanothiophen-hydrochlorid 5-Aminomethyl-2-cyanofuran-hydrochlorid eingesetzt wurde.

### Beispiel 12:

N-(Hydroxycarbonyl-methylen)-(D)-cyclohexylglycyl-3,4-dehydroprolyl-5-(2-amidino)-furanylmethylamid-hydroacetat:

FAB-MS (M+H⁺) : 432

### Beispiel 13:

N-(Hydroxycarbonyl-methylen)-(D)-cyclohexylalanyl-3,4-dehydroprolyl-5-(3-amidino)-furanylmethylamid-hydroacetat:

FAB-MS (M+H⁺): 446

### Beispiel 14:

N-(Hydroxycarbonyl-methylen)-(D)-cyclohexylalanyl-3,4-dehydroprolyl-5-(2-amidino-1-methyl)pyrrolmethylamid Hydrochlorid:
a) 5-(N-Boc-Aminomethyl)-1-methylpyrrol-2-amidin Hydroacetat (1,5 g, 4,4 mmol) wurde in Isopropanol (70 ml) gelöst, mit isopropanolischer Salzsäure (5,5 M, 4,5 ml, 24,0 mmol) versetzt und 2 h auf 50°C erwärmt. Nach Abkühlen auf Raumtemperatur wurde das Lösungsmittel abrotiert und der Rückstand zu einer Lösung von t-BuO₂C-CH₂-(Boc)-(D)-Cha-Pyr-OH in DMF (50 ml) gegeben. Die Lösung wurde auf 0°C gekühlt und mit N-Methylmorpholin (1,92 ml, 17,44 mmol) versetzt. Anschließend wurde TOTU (1,18 g, 3,58 mmol) portionsweise zugegeben. Nach 45 min Rühren bei 0°C wurde das Lösungsmittel abrotiert und das Rohprodukt mittels MPLC gereinigt (RP-18, Acetonitril/Wasser). Ausbeute: 980 mg (45%).
   FAB-MS (M+H⁺): 615.
b) Das gemäß a) erhaltene Produkt (550 mg, 0,845 mmol) wurde in Dichlormethan (50 ml) gelöst und die Lösung bei 0-5°C mit HCl-Gas gesättigt. Anschließend wurde 1,5 h bei 0°C gerührt. Das Lösungsmittel wurde abrotiert und das Rohprodukt lyophilisiert. Ausbeute: 450 mg (100%).
   FAB-MS (M+H⁺): 459.

### Beispiel 15:

N-(Hydroxycarbonyl-methylen)-(D)-cyclohexylalanyl-3,4-dehydroprolyl-2-(4-amidino-1-methyl)pyrrolmethylamid Hydrochlorid wurde analog Beispiel 14 dargestellt.

FAB-MS (M+H⁺): 459.

### Beispiel 16:

N-(Hydroxycarbonyl-methylen)-(D)-cyclohexylalanyl-3,4-dehydroprolyl-4-(2-amidino-1-methyl)pyrrolmethylamid Hydrochlorid wurde analog Beispiel 14 dargestellt.

FAB-MS (M+H⁺): 459.

### Beispiel 17:

N-(tert.Butoxycarbonyl-methylen)-(N-Boc)-(D)-cyclohexylalanyl-3,4-dehydroprolyl-2-(4-amidothiocarbonyl)oxazolmethylamid Hydrochlorid:
a) t-BuO₂C-CH₂-(Boc)-(D)-Cha-Pyr-OH (2,36 g, 4,92 mmol) wurde in Dichlormethan (60 ml) gelöst. Bei -10°C wurde Diisopropylethylamin (4,3 ml, 24,59 mmol) zugetropft. Nach 5 min Rühren bei dieser Temperatur wurde 2-Aminomethyl-oxazol-4-thiocarbamid Hydrochlorid (1g, 5,16 mmol, G. Videnov et al. Angew. Chem. 1996, 108, 1604-9, die Boc-Gruppe des in dieser Literaturstelle beschriebenen N-Boc-2-Aminomethyloxazol-4-thiocarbamid wurde mit etherischer Salzsäure gespalten und das korrespondieren Hydrochlorid durch Einengen erhalten) zugegeben und anschließend eine 50%ige Lösung von Propanphosphonsäureanhydrid in Essigsäureethylester (5,06 ml, 6,39 mmol) verdünnt mit Dichlormethan (10 ml) über 20 min zugetropft. Nach 1 h Rühren bei 0°C wurde für 3 h auf RT erwärmt. Der Ansatz wurde mit Dichlormethan verdünnt, 2x mit gesättigter Natriumhydrogencarbonatlösung, 2x mit 5%iger Zitronensäurelösung und 1x mit gesättigter Natriumchloridlösung gewaschen und über Natriumsulfat getrocknet. Das Lösungsmittel wurde abrotiert und das Rohprodukt chromatographisch gereinigt (Kieselgel, Dichlormethan:Methanol = 95:5). Ausbeute: 2,5 g (82 %).
   1-H-NMR (DMSO-d₆) δ = 0.5-2.0 (m, 31H), 3.1-5.5 (m, 8H), 5.8-6.2 (m, 2H), 8.5-9.3 (m, 3H), 9.8 (sbr, 1H).
b) N-(tert.Butoxycarbonyl-methylen)-(N-Boc)-(D)-cyclohexylalanyl-3,4-dehydroprolyl-2-(4-amidino)oxazolmethylamid Hydroacetat:
   Das gemäß a) erhaltene Produkt wurde in Aceton (50 ml) gelöst, mit Methyliodid (1,97 ml, 31,29 mmol) versetzt und 2 h unter Rückfluß erhitzt. Das Lösungsmittel und das überschüssige Methyliodid wurde abrotiert. Das so erhaltene Rohprodukt wurde in Tetrahydrofuran (50 ml) gelöst, mit Ammoniumacetat (466 mg, 6,05 mmol) versetzt und 1,5 h auf 60°C erwärmt. Das Lösungsmittel wurde abrotiert und das Rohprodukt mittels eines Ionentauschers (Acetat auf polymerem Träger, Fluka 00402) ins Acetat überführt, das anschließend chromatographisch gereinigt wurde (Kieselgel, Dichlormethan:Methanol:Essigsäure = 75:20:5). Ausbeute: 2,0 g (75 %)
   FAB-MS (M+H⁺) : 603.
c) N-(Hydroxycarbonyl-methylen)-(D)-cyclohexylalanyl-3,4-dehydroprolyl-2-(4-amidino)oxazolmethylamid Hydrochlorid:
   Das gemäß b) erhaltene Produkt (1,95 g, 2,94 mmol) wurde in Dichlormethan (50 ml) gelöst und mit 4 M Salzsäure in Dioxan (3,7 ml, 14,71 mmol) versetzt. Nach 20 h Rühren bei RT wurde das Lösungsmittel abrotiert, das Rohprodukt in Wasser gelöst und lyophilisiert. Ausbeute 1,5 g (100 %).
   13-H-NMR (DMSO-d₆) δ = 168.6, 167.8, 166.2, 162.2, 156.4, 144.7, 129.6, 127.7, 125.5, 67.9, 55.0, 53.5, 45.3, 36.4, 35.7, 33.0, 32.5, 32.0, 25.7, 25.4, 25.2.

### Beispiel 18:

N-(Hydroxycarbonyl-methylen)-(D)-cyclohexylalanyl-3,4-dehydroprolyl-5-(3-amidino)-1,2,4-oxadiazolmethylamid Hydrochlorid:
a) N-(tert-Butoxycarbonyl-methylen)-(N-Boc)-(D)-cyclohexylalanyl-3,4-dehydroprolyl-5-(3-cyano)-1,2,4-oxadiazolmethylamid
   N-(t-BuO₂C-CH₂)-N-Boc-(D)-Cha-Pyr-OH (1,93 g, 4,0 mmol) wurde in Dichlormethan (65 ml) gelöst und bei -10°C mit Diisopropylethylamin (3,1 ml, 17,67 mmol) versetzt. Anschließend wurde 5-Aminomethyl-3-cyano-1,2,4-oxadiazol Hydrochlorid (645 mg, 4,0 mmol) gelöst in Dichlormethan (30 ml) zugegeben. Nach 5 min Rühren wurde eine 50 %ige Lösung von Propanphosphonsäureanhydrid in Essigsäureethylester (3,9 ml, 4,93 mmol) verdünnt mit Dichlormethan (15 ml) über 30 min zugetropft. Nach 1 h bei 0°C wurde der Ansatz mit Dichlormethan verdünnt, 2x mit gesättigter Natriumhydrogencarbonatlösung, 2x mit 5 %iger Zitronensäurelösung und 1x mit gesättigter Natriumchloridlösung gewaschen. Nach Trocknen über Natriumsulfat wurde das Lösungsmittel abrotiert und das Rohprodukt chromatographisch gereinigt (Kieselgel, Dichlormethan: Methanol = 95:5). Ausbeute: 1,55 g (71 %).
   FAB-MS (M+H⁺) : 587.
b) N-(tert.Butoxycarbonyl-methylen)-(N-Boc)-(D)-cyclohexylalanyl-3,4-dehydroprolyl-5-(3-amidino)-1,2,4-oxadiazolmethylamid Hydroacetat
   Das gemäß a) erhaltene Produkt (1,5 g, 2,56 mmol) wurde in Methanol (5 ml) gelöst und mit Acetylcystein (450 mg, 2,76 mmol) versetzt. Anschließend wurde bei 35°C Ammoniak bis zur vollständigen Umsetzung eingeleitet. Das Lösungsmittel wurde abrotiert und das Rohprodukt mittels eines Ionenaustauschers (Acetat auf polymerem Träger, Fluka 00402) ins Acetat überführt. Das so erhaltene Rohprodukt wurde chromatographisch gereinigt (RP-18, Acetonitril, Wasser). Ausbeute: 300 mg (18 %).
   FAB-MS (M+H⁺) : 604.
c) N-(Hydroxycarbonyl-methylen)-(D)-cyclohexylalanyl-3,4-dehydroprolyl-5- (3-amidino)-1,2,4-oxadiazolmethylamid Hydrochlorid
   Das gemäß b) erhaltene Produkt (300 mg, 0,45 mmol) wurde in Dichlormethan (20 ml) gelöst und bei Raumtemperatur mit 4 M Salzsäurelösung in Dioxan (0,6 ml, 2,48 mmol) versetzt. Nach 20 h Rühren bei RT wurde das Lösungsmittel abrotiert, das Produkt in Wasser gelöst und lyophilisiert. Ausbeute: 230 mg (98%).
   FAB-MS (M+H⁺) : 448.

### Beispiel 19:

N-(Hydroxycarbonyl-methylen)-(D)-cyclohexylalanyl-3,4-dehydroprolyl-5-(3-amidino-N-methyl)pyrazolmethylamid Hydrochlorid:
a) N-(tert.Butoxycarbonyl-methylen)-(N-Boc)-(D)-cyclohexylalanyl-3,4-dehydroprolyl-5-(3-amido-N-methyl)-pyrazolmethylamid
   N-(t-BuO₂C-CH₂)-N-Boc-(D)-Cha-Pyr-OH (1,25 g, 2,59 mmol) wurde in Dichlormethan (30 ml) vorgelegt. Bei -10°C wurde Diisopropylethylamin (1,95 ml, 11,16 mmol) zugetropft. Anschließend wurde eine Lösung von N-Methyl-5-aminomethyl-pyrazol-3-carbonsäureamid (0,4 g, 2,59 mmol) in Tetrahydrofuran (20 ml) zugegeben. Nach 5 min Rühren wurde eine 50 %ige Propanphosphonsäureanhydrid Essigsäureethylesterlösung (2,36 ml, 3,11 mmol) und Dichlormethan (5 ml) innerhalb von 5 min zugetropft. Nach 45 min Rühren bei 0°C wurde für 12 h auf RT erwärmt. Das Lösungsmittel wurde abrotiert, der Rückstand in Dichlormethan aufgenommen und 2x mit gesättigter Natriumhydrogencarbonatlösung, 2x mit 5%iger Zitronensäurelösung und 1x mit gesättigter Natriumchloridlösung gewaschen. Nach Trocknen über Natriumsulfat wurde das Lösungsmittel abrotiert. Das Rohprodukt wurde chromatographisch gereinigt (RP-18, Acetonitril, Wasser). Ausbeute: 220 mg (14 %). FAB-MS (M+H⁺) : 617.
b) N- (tert.Butoxycarbonyl-methylen) - (N-Boc)- (D) -cyclohexylalanyl - 3, 4- dehydroprolyl - 5 - (3-cyano-N-methyl)-pyrazolmethylamid
   Das gemäß a) erhaltene Produkt (220 mg, 0,36 mmol) wurde in Dichlormethan (15 ml) gelöst und bei -10°C mit Diisopropylethylamin (0,17 ml, 0,96 mmol) versetzt. Nach 5 min Rühren wurde eine Lösung von Trifluoressigsäureanhydrid (0,057 ml, 0,41 mmol) in Dichlormethan (1 ml) zugetropft. Nach 1 h bei 0°C wurde mit Dichlormethan verdünnt, 2x mit gesättigter Natriumhydrogencarbonatlösung, 2x mit 5 %iger Zitronensäurelösung und 1x mit gesättigter Natriumchloridlösung gewaschen. Nach Trocknen über Natriumsulfat wurde das Lösungsmittel abrotiert. Ausbeute: 180 mg (84 %).
c) N-(tert.Butoxycarbonyl-methylen)-(N-Boc)-(D)-cyclohexylalanyl-3,4-dehydroprolyl-5-(3-amidino-N-methyl)-pyrazolmethylamid Hydroacetat
   Das gemäß b) erhaltene Produkt (180 mg, 0,3 mmol) wurde in Methanol (1 ml) gelöst und mit Acetylcystein (52,8 mg, 0,32 mmol) versetzt. Anschließend wurde bei 35°C Ammoniak bis zur vollständigen Umsetzung eingeleitet. Das Lösungsmittel wurde abrotiert und das Rohprodukt mittels eines Ionenaustauschers (Acetat auf polymerem Träger, Fluka 00402) ins Acetat überführt. Das Rohprodukt wurde chromatographisch gereinigt (RP-18, Acetonitril, Wasser). Ausbeute: 50 mg (16 %).
   FAB-MS (M+H⁺): 616.
d) N-(Hydroxycarbonyl-methylen)-(D)-cyclohexylalanyl-3,4-dehydroprolyl-5-(3-amidino-N-methyl)pyrazolmethylamid Hydrochlorid
   Das gemäß c) erhaltene Produkt (50 mg, 0,081 mmol) wurde in Dichlormethan (5 ml) gelöst und mit 5 M Salzsäure in Diethylether (0,147 ml) versetzt. Nach 12 h Rühren bei RT wurde das Lösungsmittel abrotiert das Produkt in Wasser aufgenommen und lyophilisiert. Ausbeute: 40 mg (92 %).
   FAB-MS (M+H⁺): 460.

### Beispiel 20:

N-(Hydroxycarbonyl-methylen)-(D)-cyclohexylalanyl-3,4-dehydroprolyl-5-(3-amidino)-isoxazolmethylamid-hydrochlorid:

Die Darstellung erfolgte ausgehend von 5-Aminomethylisoxazol-3-carbonsäureamid und BOC-3,4-dehydroprolin. Nach Kupplung und Abspaltung der BOC-Schutzgruppe wurde der erhaltene Baustein mit N-(tert.Butoxycarbonylmethylen)-(N-Boc)-(D)-cyclohexylalanin zu N-(tert.Butoxycarbonylmethylen)-(N-Boc)-(D)-cyclohexylalanyl-3,4-dehydroprolyl-5- (3-carbamoyl)isoxazolmethylamid verknüpft. Nach der Dehydratisierung des primären Amids zur Nitrilfunktion analog Beispiel 4 erfolgte die Amidinbildung wie nachfolgend beschrieben.

N-(tert.Butoxycarbonylmethylen)-(N-Boc)-(D)-cyclohexylalanyl-3 4-dehydroprolyl-5-(3-amidino)-isoxazolmethylamid-hydroacetat:

1,75 g (3,0 mmol) N-(tert.Butoxycarbonylmethylen)-(N-Boc)-(D)-cyclohexylalanyl-3,4-dehydroprolyl-5-(3-cyano)-isoxazolmethylamid wurden in 10 ml Methanol gelöst, mit 0,54 g (3,28 mmol) N-Acetylcystein versetzt und unter Durchleiten von gasförmigem Ammoniak 4 h unter Rückfluß erhitzt. Die Abtrennung von N-Acetylcystein, die Reinigung des Produktes und die Überführung in das AcetatSalz erfolgte mittels MPL-Chromatographie (RP-18, Acetonitril/Wasser/0,1 m Essigsäure). Nach Gefriertrocknung wurden 1,39 g als weißes Pulver erhalten (70 % der Theorie).

Die Entschützung des gereinigten Produktes mit etherischer Salzsäure in Methylenchlorid ergab die Titelverbindungerbindung

FAB-MS (M+H⁺) : 448.

### Beispiel 21:

N-(Hydroxycarbonyl-methylen)-(D)-cyclohexylalanyl-3,4-dehydroprolyl-2-(4-amidino)-thiazolmethylamid-hydroacetat

FAB-MS (M+H⁺) : 463.

Die Darstellung erfolgte analog Beispiel 1, wobei von den Edukten 2-Aminomethyl-thiazol-4-thiocarboxamid und N-Boc-N-(tert.butyloxycarbonylmethylen)-(D)-cyclohexylalanyl-3,4-dehydroprolin ausgegangen wurde.

### Beispiel 22:

N-(Hydroxycarbonyl-methylen)-(D)-cyclohexylglycyl-3,4-dehydroprolyl-2-(4-amidino)-thiazolmethylamid-hydrochlorid

FAB-MS (M+H⁺) : 449.

### Beispiel 23:

N-(Hydroxycarbonyl-methylen)-(D)-cyclohexylalanyl-3,4-dehydroprolyl-4-(2-amidino)-thiazolmethylamid-hydroacetat

FAB-MS (M+H⁺) : 463.

Die Darstellung erfolgte analog Beispiel 1, wobei von den Edukten 4-Aminomethyl-thiazol-2-thiocarboxamid und N-(tert.butoxycarbonyl-methylen)-N-Boc-(D)-cyclohexylalanyl-3,4-dehydroprolin ausgegangen wurde.

### Beispiel 24:

N-(Hydroxycarbonyl-methylen)-(D)-cyclohexylglycyl-3,4-dehydroprolyl-4-(2-amidino)-thiazolmethylamid-hydroacetat

FAB-MS (M+H⁺): 449.

Die Darstellung erfolgte analog Beispiel 1, wobei von den Edukten 4-Aminomethyl-thiazol-2-thiocarboxamid und N-(tert.butoxycarbonyl-methylen)-N-Boc-(D)-cyclohexylglycyl-3,4-dehydroprolin ausgegangen wurde.

### Beispiel 25:

N-(Hydroxycarbonyl-methylen)-(D)-cyclohexylalanyl-3,4-dehydroprolyl-2-(5-amidino)-thiazolmethylamid-hydroacetat

FAB-MS (M+H⁺): 463.

Die Darstellung erfolgte analog Beispiel 21

### Beispiel 26:

N-(Hydroxycarbonyl-methylen)-(D)-cyclohexylglycyl-3,4-dehydroprolyl-2-(5-amidino)-thiazolmethylamid-hydroacetat

FAB-MS (M+H⁺) : 449.

Die Darstellung erfolgte analog Beispiel 22.

### Beispiel 27:

N-(Hydroxycarbonyl-methylen)-(D)-cyclohexylalanyl-3,4-dehydroprolyl-5-(2-amidino)-thiazolmethylamid-hydroacetat

FAB-MS (M+H⁺) : 463.

Die Darstellung erfolgte analog Beispiel 23.

### Beispiel 28:

N-(Hydroxycarbonyl-methylen)-(D)-cyclohexylglycyl-3,4-dehydroprolyl-5-(2-amidino)-thiazolmethylamid-hydroacetat

FAB-MS (M+H⁺) : 449.

Die Darstellung erfolgte analog Beispiel 24.

## Patentansprüche

1. Verbindungen der Formel I 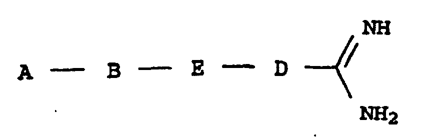 worin A, B, D und B folgende Bedeutung besitzen:
A: 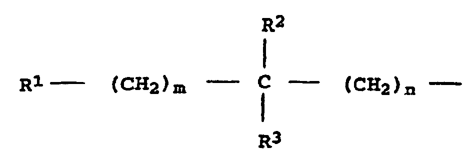 worin
m 0, 1 oder 2,
n 0, 1 oder 2,
R¹ HOOC-, C₁₋₆-Alkyl-OOC- oder -OH,
R² H-, C₁₋₄-Alkyl- oder R¹-(CH₂)ₘ-,
R³ H- oder C₁₋₄-Alkyl-,
darstellen,
B: 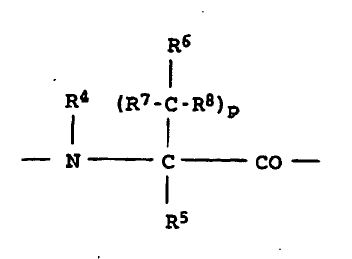 worin
R⁴ H-, C₁₋₄-Alkyl- oder R¹-(CH₂)ₘ- (wobei R¹ und m die oben angegebene Bedeutung besitzen),
p 0 oder 1,
R⁵ H- oder C₁₋₄-Alkyl-,
R⁶ H-, C₁₋₈-Alkyl-, Phenyl-, welches bis zu drei gleiche oder verschiedene Reste der Gruppe C₁₋₄-Alkyl-, CF₃-, C₁₋₄-Alkoxy-, F- oder Cl- tragen kann, C₃₋₈-Cycloalkyl-, welches bis zu vier gleiche oder verschiedene C₁₋₄-Alkylreste tragen kann oder wobei eine oder zwei C-C-Einfachbindungen im Ring durch eine C=C-Doppelbindung ersetzt sein können, oder ein Phenylring ankondensiert sein kann, C₇-C₁₂-Bicycloalkyl- oder C₁₀-Tricycloalkyl- oder
R⁴ und R⁶ zusammen eine Ethylen- oder Propylengruppe,
R⁷ H, C₁₋₈-Alkyl-, Phenyl-, welches bis zu drei gleiche oder verschiedene Reste der Gruppe C₁₋₄-Alkyl-, CF₃-, C₁₋₄-Alkoxy-, F- oder Cl- tragen kann, C₃₋₈-Cycloalkyl-, welches bis zu vier gleiche oder verschiedene C₁₋₄-Alkylreste tragen kann,
R⁸ H oder C₁₋₄-Alkyl,
E: 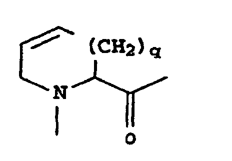 q = 0 oder 1
D: 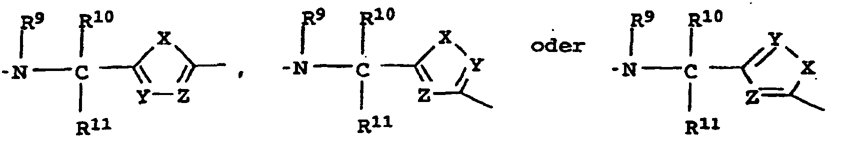 worin
R⁹ H- oder C₁₋₃-Alkyl-,
R¹⁰ H- oder C₁₋₄-Alkyl-,
R¹¹ H- oder C₁₋₄-Alkyl-,
X O, S, -NR¹² (R¹² = H-, C₁₋₆-Alkyl-),
Y -N= oder -CR¹³= (R¹³ = H-, C₁₋₄-Alkyl-), Cl, CF₃,
Z -N= oder -CR¹³= bedeuten,
sowie deren Salze mit physiologisch verträglichen Säuren.

2. Verbindungen der Formel I gemäß Anspruch 1 zur Verwendung bei der Bekämpfung von Krankheiten.

3. Verwendung der Verbindungen der Formel I gemäß Anspruch 1 zur Herstellung von Arzneimitteln gegen:
• Krankheiten, deren Pathomechanismus direkt oder indirekt auf der proteolytischen Wirkung von Thrombin beruht,
• Krankheiten, deren Pathomechanismus auf der thrombinabhängigen Aktivierung von Rezeptoren und Signaltransduktionen beruht,
• Krankheiten, die mit Stimulation oder Inhibition von Genexpressionen in Körperzellen einhergehen,
• Krankheiten, die auf der mitogenen Wirkung von Thrombin beruhen,
• Krankheiten, die auf einer thrombinabhängigen Kontraktilitäts- und Permeabilitatsveränderung von Epithelzellen beruhen,
• thrombinabhängige, thromboembolische Ereignisse,
• disseminierte intravasale Koagulation,
• Reokklusion und zur Verkürzung der Reperfusionszeit bei Komedikation mit Thrombolytika,
• das Auftreten von früher Reokklusion und später Restenosierung nach PTCA,
• die thrombinabhängige Proliferation von Glattmuskelzellen,
• die Akkumulation aktiven Thrombins im ZNS,
• das Tumorwachstum sowie gegen die Adhäsion und Metastasierung von Tumorzellen.

4. Verwendung der Verbindungen der Formel I gemäß Anspruch 1 zur Herstellung von Arzneimitteln gegen:
• Krankheiten, deren Pathomechanismus direkt oder indirekt auf der proteolytischen Wirkung von Kininogenasen, insbesondere Kallikrein beruht,
• Entzündungskrankheiten wie Asthma, Pankreatitis, Rhinitis, Arthritis, Urticaria und anderen inneren Krankheiten, bei denen Kallikrein eine Rolle spielt.

5. Verbindungen der Formel I gemäß Anspruch 1 zur Beschichtung von Oberflächen.

6. Verbindungen enthaltend ein strukturelles Fragment der Formel 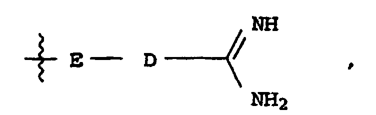 worin E und D die in Anspruch 1 angegebene Bedeutung besitzen.

7. Verbindungen der. Formel IIa und IIb
A ― B ― E ― D ― CN IIa,,
A ― B ― E ― D ― CSNH₂ IIb,
worin die Reste A, B, E und D die in Anspruch 1 angegebene Bedeutung besitzen.

## Claims

1. Compounds of the formula I 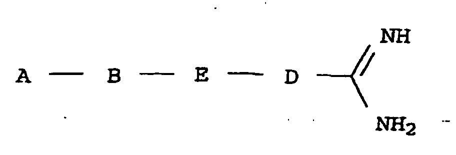 where A, B, D and E have the following meanings:
A: 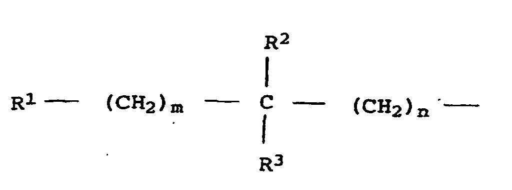 where
m is 0, 1 or 2,
n is 0, 1 or 2,
R¹ is HOOC-, C₁₋₆-alkyl-OOC- or -OH,
R² is H, C₁₋₄-alkyl or R¹- (CH₂)ₘ-,
R³ is H or C₁₋₄-alkyl,
B: 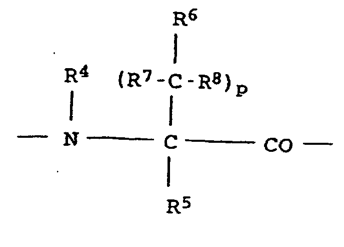 where
R⁴ is H, C₁₋₄-alkyl or R¹-(CH₂)ₘ- (where R¹ and m have the above-mentioned meanings),
p is 0 or 1,
R⁵ is H or C₁₋₄-alkyl,
R⁶ is H, C₁₋₈-alkyl, phenyl which may carry up to three identical or different radicals from the group C₁₋₄-alkyl, CF₃, C₁₋₄-alkoxy, F and Cl, or C₃₋₈-cycloalkyl which may carry up to four identical or different C₁₋₄-alkyl radicals, or where one or two C-C single bonds in the ring can be replaced by a C=C double bond, or a phenyl ring can be fused on, C₇₋₁₂-bicycloalkyl or C₁₀-tricycloalkyl or
R⁴ and R⁶ together are an ethylene or propylene group,
R⁷ is H, C₁₋₈-alkyl, phenyl which may carry up to three identical or different radicals from the group C₁₋₄-alkyl, CF₃, C₁₋₄-alkoxy, F and Cl, or C₃₋₈-cycloalkyl which may carry up to four identical or different C₁₋₄-alkyl radicals,
R⁸ is H or C₁₋₄-alkyl,
E: 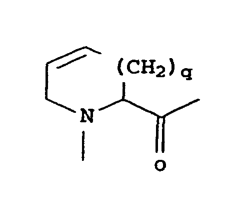 q = 0 or 1,
D: 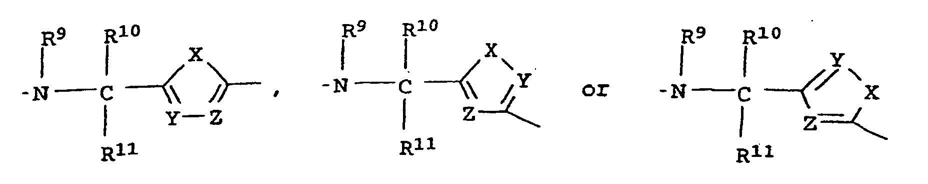 where
R⁹ is H or C₁₋₃-alkyl,
R¹⁰ is H or C₁₋₄-alkyl,
R¹¹ is H or C₁₋₄-alkyl,
X is O, S, -NR¹² (R¹² = H, C₁₋₆-alkyl),
Y is -N= or -CR¹³= (R¹³ = H, C₁₋₄-alkyl), Cl, CF₃,
Z is -N= or -CR¹³=,
and the salts thereof with physiologically tolerable acids.

2. Compounds of the formula I as claimed in claim 1 for use for controlling diseases.

3. The use of the compounds of the formula I as claimed in claim 1 for producing drugs against:
• diseases whose pathogenetic mechanism derives directly or indirectly from the proteolytic effect of thrombin,
• diseases whose pathogenetic mechanism derives from the thrombin-dependent activation of receptors and signal transductions,
• diseases associated with stimulation or inhibition of the expression of genes in body cells,
• diseases deriving from the mitogenic effect of thrombin,
• diseases deriving from a thrombin-dependent change in the contractility and permeability of epithelial cells,
• thrombin-dependent thromboembolic events,
• disseminated intravascular coagulation,
• reocclusion and for reducing the reperfusion time on comedication with thrombolytics,
• the occurrence of early reocclusion and late restenosis after PTCA,
• the thrombin-dependent proliferation of smooth muscle cells,
• the accumulation of active thrombin in the CNS,
• tumour growth and also against the adhesion and metastasis of tumour cells.

4. The use of compounds of the formula I as claimed in claim 1 for producing drugs against:
• diseases whose pathogenetic mechanism derives directly or indirectly from the proteolytic effect of kininogenases, especially kallikrein,
• inflammatory diseases such as asthma, pancreatitis, rhinitis, arthritis, urticaria and other internal diseases in which kallikrein is involved.

5. Compounds of the formula I as claimed in claim 1 for coating surfaces.

6. Compounds comprising a structural fragment of the formula 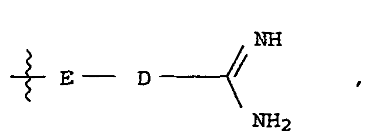 where E and D have the meanings indicated in claim 1.

7. Compounds of the formula IIa and IIb
A ― B ― E ― D ― CN IIa,
A ― B ― E ― D ― CSNH₂ IIb,
where the radicals A, B, E and D have the meanings indicated in claim 1.

## Revendications

1. Composés de formule I 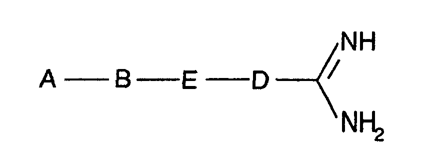 où A, B, D et E possèdent la signification suivante :
A: 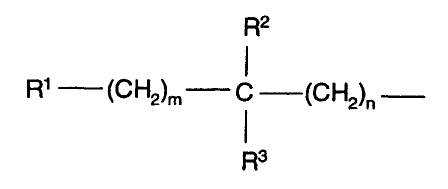 où
m représente 0, 1 ou 2
n représente 0, 1 ou 2
R¹ représente HOOC-, alkyle en C₁₋₆-OOC- ou -OH,
R² représente H-, alkyle en C₁₋₄ ou R¹-(CH₂)ₘ,
R³ représente H- ou alkyle en C₁₋₄,
B: 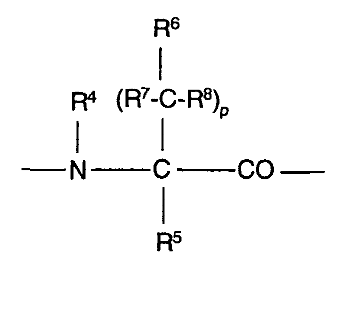 où
R⁴ représente H-, alkyle en C₁₋₄ ou R¹-(CH₂)ₘ- (où R¹ et m possèdent la signification indiquée ci-dessus),
p représente 0 ou 1,
R⁵ représente H- ou alkyle en C₁₋₄,
R⁶ représente H-, alkyle en C₁₋₈, phényle-, qui peut porter jusqu'à 3 restes identiques ou différents du groupe alkyle en C₁₋₄, CF₃-, alcoxy en C₁₋₄, F- ou Cl-, cycloalkyle en C₃₋₈, qui peut porter jusqu'à 4 restes alkyle en C₁₋₄ identiques ou différents ou bien où une ou deux simples liaisons C-C dans le cycle peuvent être remplacées par une double liaison C=C, ou bien un cycle phényle peut être condensé, bicycloalkyle en C₇-C₁₂ ou tricycloalkyle en C₁₀ ou bien
R⁴ et R⁶ représentent ensemble un groupe éthylène ou propylène,
R⁷ représente H, alkyle en C₁₋₈, phényle, qui peut porter jusqu'à 3 restes identiques ou différents du groupe alkyle en C₁₋₄, CF₃-, alcoxy en C₁₋₄, F- ou CI-, cycloalkyle en C₃₋₈, qui peut porter jusqu'à 4 restes alkyle en C₁₋₄ identiques ou différents,
R⁸ représente H ou alkyle en C₁₋₄,
E: 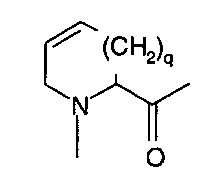 q = 0 ou 1
D: 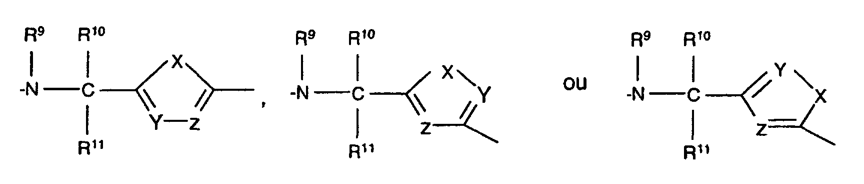 où
R⁹ représente H- ou alkyle en C₁₋₃,
R¹⁰ représente H- ou alkyle en C₁₋₄,
R¹¹ représente H- ou alkyle en C₁₋₄,
X représente O, S, -NR¹² (R¹² = H-, alkyle en C₁₋₆),
Y représente -N= ou -CR¹³= (R¹³ = H-, alkyle en C₁₋₄), Cl, CF₃,
Z représente -N= ou -CR¹³=,
ainsi que leurs sels avec des acides physiologiquement acceptables.

2. Composés de formule I selon la revendication 1 destinés à être utilisés dans la lutte contre des maladies.

3. Utilisation des composés de formule I selon la revendication 1 pour la production de médicaments contre :
- les maladies dont le mécanisme pathologique repose directement ou indirectement sur l'effet protéolytique de la thrombine,
- les maladies dont le mécanisme pathologique repose sur l'activation dépendante de la thrombine de récepteurs et de transductions de signaux,
- les maladies qui s'accompagnent d'une stimulation ou d'une inhibition d'expressions géniques dans des cellules corporelles,
- les maladies qui reposent sur l'effet mitogène de la thrombine,
- les maladies qui reposent sur une modification dépendante de la thrombine de la contractilité et de la perméabilité des cellules épithéliales,
- les événements thrombo-emboliques dépendants de la thrombine,
- la coagulation intravasculaire disséminée,
- la réocclusion et pour raccourcir la durée de reperfusion dans le cas d'une co-médication avec des thrombolytiques,
- la survenue d'une réocclusion plus tôt et d'une resténose plus tard après une ACTP,
- la prolifération dépendant de la thrombine de cellules de muscles lisses,
- l'accumulation de thrombines actives dans le SNC,
- la croissance tumorale ainsi que contre l'adhésion et la formation de métastases de cellules tumorales.

4. Utilisation des composés de formule I selon la revendication 1 pour la production de médicaments contre :
- les maladies dont le mécanisme pathologique repose directement ou indirectement sur l'effet protéolytique de kininogénases, en particulier de la kallikréine,
- les maladies inflammatoires comme l'asthme, la pancréatite, la rhinite, l'arthrite, l'urticaire et d'autres maladies internes dans lesquelles la kallikréine joue un rôle.

5. Composés de formule I selon la revendication 1 destinés à revêtir des surfaces.

6. Composés contenant un fragment structural de formule 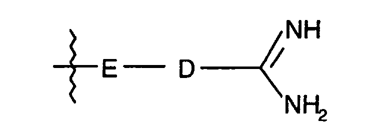 où E et D possèdent la signification indiquée dans la revendication 1.

7. Composés des formules IIa et IIb
A ― B ― E ― D ― CN IIa,,
A ― B ― E ― D ― CSNH₂ IIb,
où les restes A, B, E et D possèdent la signification indiquée dans la revendication 1.
